(19) Europäisches Patentamt
European Patent Office
Office européen des brevets

(11) Veröffentlichungsnummer: **0 403 885 A1**

(12) **EUROPÄISCHE PATENTANMELDUNG**

(21) Anmeldenummer: **90110764.9**

(22) Anmeldetag: **07.06.90**

(51) Int. Cl.5: **C07D 333/70, C07D 495/04, A61K 31/38, A61K 31/44, C07C 323/62**

(30) Priorität: **20.06.89 DE 3920087**
**19.09.89 DE 3931157**

(43) Veröffentlichungstag der Anmeldung:
**27.12.90 Patentblatt 90/52**

(84) Benannte Vertragsstaaten:
**AT BE CH DE DK ES FR GB GR IT LI NL SE**

(71) Anmelder: **BAYER AG**

**D-5090 Leverkusen 1 Bayerwerk(DE)**

(72) Erfinder: **Müller, Nikolaus, Dr.**
**Knipprather Strasse 98**
**D-4019 Monheim(DE)**
Erfinder: **Hallenbach, Werner, Dr.**
**Lichtenberger Strasse 68**
**D-4019 Monheim(DE)**
Erfinder: **Harder, Achim, Dr. Dr.**
**Auf dem Bruch 49**
**D-5090 Leverkusen(DE)**
Erfinder: **Lindner, Werner, Dr.**
**Märchenstrasse 39**
**D-5000 Köln 80(DE)**

(54) **Verwendung von 3-Hydroxybenzothiophenen zur Bekämpfung von Endoparasiten, neue 3-Hydroxybenzothiophene und Verfahren zu ihrer Herstellung.**

(57) Die vorliegende Erfindung betrifft die Verwendung von 3-Hydroxybenzothiophenen der Formel I

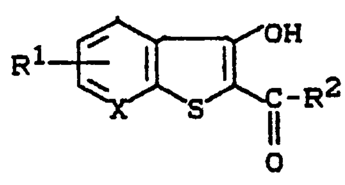

(I)

in welcher
$R^1$ für einen oder mehrere, gleiche oder verschiedene der Reste Wasserstoff, Alkyl, Alkoxy, Alkylthio, Halogenalkyl, Halogenalkoxy, Halogenalkylthio, Alkylendioxy, Halogenalkylendioxy, Halogen, CN, $NO_2$, $NH_2$, Alkylamino, Dialkylamino, Alkylcarbonyl Carbalkoxy, Alkylsulfonyl, Arylsulfonyl, Sulfamoyl, Alkylsulfamoyl, Dialkylsulfamoyl, Aryl Aryloxy, Arylthio, die ihrerseits wieder substituiert sein können, steht,
$R^2$ für gegebenenfalls substituiertes Alkoxy, Cycloalkoxy oder den Rest-$NR^3R^4$ steht,
X für CH oder N steht,
$R^3$ für Wasserstoff oder Alkyl steht,
$R^4$ für carbocyclische oder heterocyclische aromatische Reste steht,
zur Bekämpfung von Endoparasiten in der Medizin und Tiermedizin, neue Verbindungen der Formel I und Verfahren zu ihrer Herstellung.

EP 0 403 885 A1

# Verwendung von 3-Hydroxybenzothiophenen zur Bekämpfung von Endoparasiten, neue 3-Hydroxybenzothiophene und Verfahren zu ihrer Herstellung

Die vorliegende Erfindung betrifft die Verwendung von 3-Hydroxybenzothiophenen zur Bekämpfung von Endoparasiten, neue 3-Hydroxybenzothiophene und Verfahren zu ihrer Herstellung.

Substituierte Hydroxybenzothiophene sind bereits bekannt. Es ist jedoch nichts über ihre Verwendung gegen Endoparasiten bekannt (DE-OS 1 937 514, GB-PS 2 193 961, DOS 2 258 036).

Die vorliegende Erfindung betrifft

1. die Verwendung von 3-Hydroxybenzothiophenen der Formel (I)

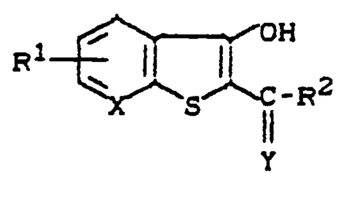

(I)

in welcher
X für =CH- oder =N- steht,
Y für =O oder =NH steht,
$R^1$ für einen oder mehrere, gleiche oder verschiedene der Reste Wasserstoff, Alkyl, Alkoxy, Alkylthio, Halogenalkyl, Halogenalkoxy, Halogenalkylthio, Alkylendioxy, Halogenalkylendioxy, Halogen, CN, $NO_2$, $NH_2$, Alkylamino, Dialkylamino, Alkylcarbonyl, Carbalkoxy, Alkylsulfonyl, Arylsulfonyl, Sulfamoyl, Alkylsulfamoyl, Dialkylsulfamoyl, Aryl, Aryloxy, Arylthio, die ihrerseits wieder substituiert sein können, steht,
$R^2$ für gegebenenfalls substituiertes Alkoxy, Cycloalkoxy oder den Rest-$NR^3R^4$ steht,
$R^3$ für Wasserstoff oder Alkyl steht,
$R^4$ für Alkyl, Aralkyl oder carbocyclische, heterocyclische, aromatische Reste oder den Rest -$COOR^5$ steht,
$R^3$ und $R^4$ gemeinsam mit dem angrenzenden Stickstoffatom für einen 5- oder 6-gliedrigen Heterocyclus stehen, der als weitere Heteroatome O oder N enthalten kann und gegebenenfalls durch $C_1$-$C_4$-Alkyl, $C_1$-$C_4$-Hydroxyalkyl, $C_1$-$C_4$-Halogenalkyl, $C_1$-$C_4$-Alkoxyalkyl, gegebenenfalls substituiertes Aryl substituiert ist
$R^5$ für Alkyl, Cycloalkyl, Aralkyl oder Aryl, die ihrerseits wieder substituiert sein können, steht,
zur Bekämpfung von Endoparasiten in der Medizin und Tiermedizin.

Die Verbindungen der Formel I sind teilweise bekannt und lassen sich analog zu bekannten Verfahren herstellen.

2. Neue 3-Hydroxybenzothiophene der Formel (I)

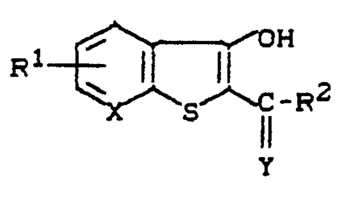

(I)

in welcher
X für =N- steht,
Y für =O oder =NH steht,
$R^1$ für einen oder mehrere, gleiche oder verschiedene der Reste Wasserstoff, Alkyl, Alkoxy, Alkylthio, Halogenalkyl, Halogenalkoxy, Halogenalkylthio, Alkylendioxy, Halogenalkylendioxy, Halogen, CN, $NO_2$, $NH_2$, Alkylamino, Dialkylamino, Alkylcarbonyl, Carbalkoxy, Alkylsulfonyl, Arylsulfonyl, Sulfamoyl, Alkylsulfamoyl, Dialkylsulfamoyl, Aryl, Aryloxy, Arylthio, die ihrerseits wieder substituiert sein können, steht,
$R^2$ für gegebenenfalls substituiertes $C_{2-6}$-Alkoxy, Cycloalkoxy oder den Rest -$NR^3R^4$ steht,
$R^3$ für Wasserstoff oder Alkyl steht,
$R^4$ für Alkyl, Aralkyl, carbocyclische oder heterocyclische aromatische Reste steht,
$R^3$ und $R^4$ gemeinsam mit dem angrenzenden Stickstoffatom für einen 5- oder 6-gliedrigen Heterocyclus stehen, der als weitere Heteroatome O oder N enthalten kann und gegebenenfalls durch $C_1$-$C_4$-Alkyl, $C_1$-$C_4$-Hydroxyalkyl, $C_1$-$C_4$-Halogenalkyl, $C_1$-$C_4$-Alkoxyalkyl, gegebenenfalls substituiertes Aryl substituiert ist.

3. Verfahren zur Herstellung der neuen 3-Hydroxybenzothiophene der Formel (I)

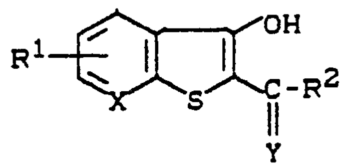

(I)

in welcher
X für =N- steht,
Y für =O oder =NH steht,
$R^1$ für einen oder mehrere, gleiche oder verschiedene der Reste Wasserstoff, Alkyl, Alkoxy, Alkylthio, Halogenalkyl, Halogenalkoxy, Halogenalkylthio, Alkylendioxy, Halogenalkylendioxy, Halogen, CN, $NO_2$, $NH_2$, Alkylamino, Dialkylamino, Alkylcarbonyl, Carbalkoxy, Alkylsulfonyl, Arylsulfonyl, Sulfamoyl, Alkylsulfamoyl, Dialkylsulfamoyl, Aryl, Aryloxy, Arylthio, die ihrerseits wieder substituiert sein können, steht,
$R^2$ für gegebenenfalls substituiertes $C_{2-6}$-Alkoxy, Cycloalkoxy oder den Rest $-NR^3R^4$ steht,
$R^3$ für Wasserstoff oder Alkyl steht,
$R^4$ für Alkyl, Aralkyl, carbocyclische oder heterocyclische aromatische Reste steht,
$R^3$ und $R^4$ gemeinsam mit dem angrenzenden Stickstoffatom für einen 5- oder 6-gliedrigen Heterocyclus stehen, der als weitere Heteroatome O oder N enthalten kann und gegebenenfalls durch $C_1$-$C_4$-Alkyl, $C_1$-$C_4$-Hydroxyalkyl, $C_1$-$C_4$-Halogenalkyl, $C_1$-$C_4$-Alkoxyalkyl gegebenenfalls substituiertes Aryl substituiert ist,
dadurch gekennzeichnet, daß man

a) Verbindungen der Formel (II)

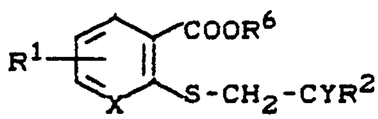

(II)

in welcher
X, Y, $R^1$, $R^2$ die oben angegebene Bedeutung haben,
$R^6$ für $C_{1-4}$-Alkyl steht,
in Gegenwart einer Base erhitzt, oder indem man

b) Verbindungen der Formel (III)

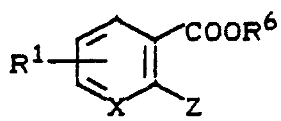

(III)

in welcher
X, $R^1$, $R^6$ die oben angegebene Bedeutung besitzen,
Y für O steht,
Z für Halogen steht,
mit Verbindungen der Formel (IV)

$$\text{H-S-CH}_2\text{-}\underset{\underset{\displaystyle \text{O}}{\|}}{\text{C}}\text{-R}^2 \qquad \text{(IV)}$$

in welcher
$R^2$ die oben angegebene Bedeutung besitzt,
in Gegenwart einer Base umsetzt.

4. Neue 3-Hydroxybenzothiophene der Formel (V)

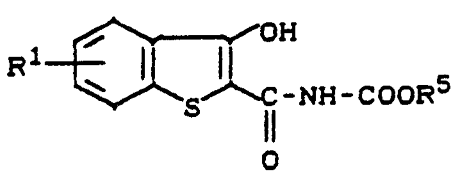

(V)

in welcher
$R^1$ für einen oder mehrere, gleiche oder verschiedene der Reste Wasserstoff, Alkyl, Alkoxy, Alkylthio, Halogenalkyl, Halogenalkoxy, Halogenalkylthio, Alkylendioxy, Halogenalkylendioxy, Halogen, CN, $NO_2$, $NH_2$, Alkylamino, Dialkylamino, Alkylcarbonyl, Carbalkoxy, Alkylsulfonyl, Arylsulfonyl, Sulfamoyl, Alkylsulfamoyl, Dialkylsulfamoyl, Aryl, Aryloxy, Arylthio, die ihrerseits wieder substituiert sein können, steht,
$R^5$ für Alkyl, Cycloalkyl, Aralkyl oder Aryl, die ihrerseits wieder substituiert sein können, steht.

5. Verfahren zur Herstellung der neuen 3-Hydroxybenzothiophene der Formel (V)

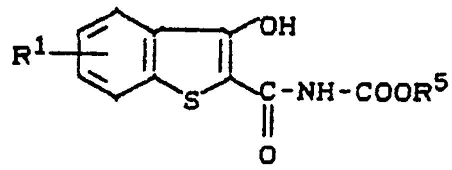
(V)

in welcher
$R^1$ für einen oder mehrere, gleiche oder verschiedene der Reste Wasserstoff, Alkyl, Alkoxy, Alkylthio, Halogenalkyl, Halogenalkoxy, Halogenalkylthio, Alkylendioxy, Halogenalkylendioxy, Halogen, CN, $NO_2$, $NH_2$, Alkylamino, Dialkylamino, Alkylcarbonyl, Carbalkoxy, Alkylsulfonyl, Arylsulfonyl, Sulfamoyl, Alkylsulfamoyl, Dialkylsulfamoyl, Aryl, Aryloxy, Arylthio, die ihrerseits wieder substituiert sein können, steht,
$R^5$ für Alkyl, Cycloalkyl, Aralkyl oder Aryl, die ihrerseits wieder substituiert sein können, steht,
dadurch gekennzeichnet, daß man
Verbindungen der Formel (VI)

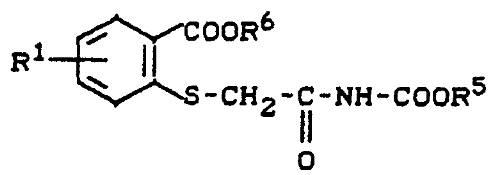
(VI)

in welcher
$R^1$ und $R^5$ die oben angegebene Bedeutung haben,
$R^6$ für $C_1$-$C_4$-Alkyl steht,
in Gegenwart einer Base erhitzt.

6. Neue Verbindungen der Formel (VI)

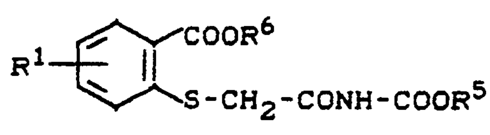
(VI)

in welcher
$R^1$, $R^5$, $R^6$ die unter Punkt 5 angegebene Bedeutung haben.

7. Verfahren zur Herstellung der Verbindungen der Formel (VI) gemäß Punkt 6, dadurch gekennzeichnet, daß man Säurechloride der Formel (VII)

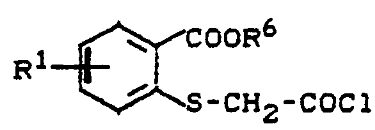
(VII)

in welcher
$R^1$, $R^6$ die unter Punkt 6 angegebene Bedeutung haben,
mit Urethanen der Formel (VIII)
$H_2N$-$COOR^5$ (VIII)
in welcher

$R^5$ die unter Punkt 6 angegebene Bedeutung hat, umsetzt.

8. Verbindungen der Formel (VII)

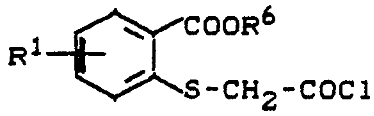

(VII)

in welcher
$R^1$ und $R^6$ die unter Punkt 6 angegebene Bedeutung haben.

9. Verfahren zur Herstellung der Verbindungen der Formel VII gemäß Punkt 8, dadurch gekennzeichnet, daß man 3-(2-Carboalkoxyphenyl)-thioglycolsäuren der Formel (IX)

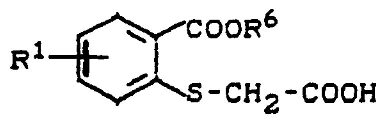

(IX)

in welcher
$R^1$ und $R^6$ die oben angegebene Bedeutung haben,
mit Thionylchlorid, Phosphortrichlorid oder Phosgen umsetzt.

Die Verbindungen der Formel (I) eignen sich hervorragend zur Bekämpfung von Endoparasiten, besonders auf dem Gebiet der Veterinärmedizin.

Bevorzugt sind Verbindungen der Formel I,
in welcher
$R^1$ für Alkyl mit vorzugsweise 1 bis 4, insbesondere 1 oder 2 Kohlenstoffatomen, wie Methyl, Ethyl, n-und i.-Propyl und n.-, i.-, s.- und t.-Butyl; Alkoxy mit vorzugsweise 1 bis 4, insbesondere 1 oder 2 Kohlenstoffatomen, wie Methoxy, Ethoxy, n.-und i.-Propyloxy und n.-, i.-, s.- und t.-Butyloxy; Alkylthio mit vorzugsweise 1 bis 4, insbesondere 1 oder 2 Kohlenstoffatomen, wie Methylthio, Ethylthio, n.-und i.-Propylthio und n.-, i.-, s.- und t.-Butylthio; Halogenalkyl mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome, vorzugsweise Fluor, Chlor oder Brom, insbesondere Fluor stehen, wie Trifluormethyl, Fluor-, Chlorethyl; Halogenalkoxy mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome vorzugsweise Fluor, Chlor, Brom, insbesondere Fluor stehen wie Trifluormethoxy; Halogenalkylthio mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome vorzugsweise Fluor, Chlor, Brom, insbesondere Fluor stehen, wie Trifluormethylthio; im Falle von Phenyl, für Alkylendioxy mit vorzugsweise 1 oder 2 Kohlenstoffatomen wie Methylendioxy oder Ethylendioxy; im Falle von Phenyl für halogensubstituiertes Alkylendioxy mit vorzugsweise 1 oder 2 Kohlenstoffatomen und vorzugsweise 1 bis 4, insbesondere 2 bis 3 Halogenatomen, wobei die Halogenatome gleich oder verschieden sind und als Halogenatome vorzugsweise Fluor oder Chlor, insbesondere Fluor stehen wie Difluormethylendioxy, Trifluorethylendioxy, Tetrafluorethylendioxy. Weitere Substituenten sind Halogen, vorzugsweise Fluor, Chlor, Brom und Jod, insbesondere Chlor und Brom; Cyano; Nitro; Dialkylamino mit vorzugsweise 1 bis 4, insbesondere oder 2 Kohlenstoffatomen je Alkylgruppe, wie Dimethylamino, Diethylamino, Methyl-n.-butylamino; Alkylcarbonyl mit vorzugsweise 2-4 Kohlenstoffatomen; Carbalkoxy mit vorzugsweise 2 bis 4, insbesondere 2 oder 3 Kohlenstoffatomen, wie Carbomethoxy und Carboethoxy; Alkylsulfonyl mit vorzugsweise 1 bis 4, insbesondere 1 oder 2 Kohlenstoffatomen, wie Methylsulfonyl und Ethylsulfonyl; Arylsulfonyl mit vorzugsweise 6 oder 10 Arylkohlenstoffatomen, wie Phenylsulfonyl; Phenyl, Naphthyl, Phenoxy, Naphthoxy, Phenylthio, Naphthylthio, die ihrerseits wieder substituiert sein können,
$R^2$ für $C_{1-6}$-Alkoxy steht, das gegebenenfalls substituiert ist durch Phenyl, das gegebenenfalls seinerseits durch einen der bei $R^1$ angegebenen Reste substituiert ist, für $C_{3-7}$-Cycloalkoxy, oder den Rest $-NR^3R^4$ steht, wobei
$R^3$ für Wasserstoff oder Alkyl steht,
$R^4$ für $C_{1-4}$-Alkyl, Benzyl oder Phenyl steht, die gegebenenfalls durch einen der bei $R^1$ angegebenen Reste

substituiert sind oder für einen Rest der Formel -COOR$^5$ steht,

R$^3$ und R$^4$ gemeinsam mit dem angrenzenden Stickstoffatom für einen 5- oder 6-gliedrigen Heterocyclus stehen, der als weitere Heteroatome O oder N enthalten kann und gegebenenfalls durch $C_1$-$C_4$-Alkyl, $C_1$-$C_4$-Hydroxyalkyl, $C_1$-$C_4$-Halogenalkyl, $C_1$-$C_4$-Alkoxyalkyl gegebenenfalls substituiertes Phenyl, Naphthyl, Pyridyl substituiert ist.

R$^5$ für $C_1$-$C_4$-Alkyl, $C_4$-$C_7$-Cycloalkyl, Aralkyl, Aryl steht, die ihrerseits durch einen der unter R$^1$ aufgeführten Reste substituiert sein können,

X für =CH- oder =N- steht,

Y für =O oder =NH steht.

Besonders bevorzugt sind Verbindungen der Formel I,

in welcher

R$^1$ für Halogen, insbesondere Chlor oder Fluor, $C_1$-$C_4$-Alkyl wie Methyl, Ethyl, $C_{1-4}$-Alkoxy wie Methoxy oder Ethoxy, $C_{1-4}$-Halogenalkoxy wie Trifluormethoxy, $C_{1-4}$-Halogenalkylthio wie Trifluormethylthio, Phenyl, das gegebenenfalls substituiert ist, ferner für Phenyl steht, das gegebenenfalls substituiert ist durch $C_1$-$C_4$-Alkyl, insbesondere Methyl, $C_1$-$C_4$-Alkoxy, insbesondere Methoxy, Ethoxy, $C_1$-$C_4$-Halogenalkoxy, insbesondere Trifluormethoxy, Fluorchlorethoxy, $C_1$-$C_4$-Halogenalylthio, insbesondere Trifluormethylthio, Fluorchlormethylthio, $C_1$-$C_4$-Alkylthio, insbesondere Methylthio, Halogensulfonyl, insbesondere Fluorsulfonyl, Chlorsulfonyl, $C_1$-$C_4$-Alkylsulfonyl, insbesondere Methylsulfonyl, $C_1$-$C_4$-Halogenalkylsulfonyl, insbesondere Trifluormethylsulfonyl, $C_1$-$C_4$-Halogenalkyl, insbesondere Trifluormethyl, Methylendioxy oder Ethylendioxy, die gegebenenfalls durch Fluor oder Chlor substituiert sind, Halogen, insbesondere Fluor oder Chlor, $NO_2$, Phenoxy, das gegebenenfalls durch einen der oben angegebenen Reste substituiert ist,

R$^2$ für $C_{1-4}$-Alkoxy, Cyclohexyloxy, Benzyloxy, Phenylethyloxy, Phenylpropyloxy steht, wobei die Phenylreste gegebenenfalls durch einen der bei R$^1$ angegebenen besonders bevorzugten Reste substituiert sein können, oder den Rest -NR$^3$R$^4$ steht, wobei

R$^3$ für Wasserstoff steht,

R$^4$ für $C_{1-4}$-Alkyl, Benzyl oder Phenyl steht, die gegebenenfalls durch einen der bei R$^1$ angegebenen besonders bevorzugten Reste substitiert sind oder für einen Rest der Formel -COOR$^5$ steht,

R$^3$ und R$^4$ gemeinsam mit dem angrenzenden Stickstoffatom für einen der Reste Piperidino, Morpholino, Pyrolidino, N-Methylpiperazino, 2,6-Dimethylmorpholino, 2,6-Diphenylmorpholino stehen

R$^5$ für $C_{1-4}$-Alkyl, Benzyl steht,

X für =CH- oder =N- steht,

Y für O oder =NH steht.

Ganz besonders bevorzugt sind Verbindungen der Formel (I),

in welcher

R$^1$ für Halogen, insbesondere Fluor, Chlor, $NO_2$, $CF_3$, $CH_3$, $OCF_3$, $SCF_3$, $SCF_2Cl$, $OCH_3$, $OCF_2CF_2H$, $-OCF_2CHFO-$, $-O-CH_2-O$, $-O-CF_2-O$ steht,

R$^2$ für $C_{1-4}$-Alkoxy, Cyclohexyloxy, Benzyloxy, Phenylethyloxy, Phenylpropyloxy steht, wobei die Phenylreste gegebenenfalls durch Halogen wie Chlor oder $C_{1-4}$-Alkoxycarbonyl substituiert sind, oder für den Rest -NR$^3$R$^4$ steht, wobei

R$^3$ für Wasserstoff steht,

R$^4$ für $C_{1-4}$-Alkyl, Benzyl oder Phenyl steht, die gegebenenfalls durch Halogen wie Chlor, Fluor, Brom, $C_{1-4}$-Alkyl wie Methyl, $C_{1-4}$-Halogenalkyl wie Trifluormethyl, $C_{1-4}$-Alkoxy wie Methoxy, $C_{1-4}$-Alkylmercapto wie Methylmercapto, $C_{1-4}$-Halogenalkylmercapto wie Trifluormethylmercapto, $C_{1-4}$-Alkoxycarbonyl wie Methoxycarbonyl substituiert sind oder für den Rest -COOR$^5$ steht,

R$^3$ und R$^4$ gemeinsam mit dem angrenzenden Stickstoffatom für einen der Reste Piperidino, Morpholino, 2,6-Dimethylmorpholino, N-Methylpiperazino stehen,

R$^5$ für $C_{1-4}$-Alkyl, Benzyl steht,

X für =CH- oder =N- steht,

Y für O oder =NH steht.

Im einzelnen seien folgende Verbindungen der Formel (I) genannt, in welcher die Reste R$^1$, R$^2$, X die angegebene Bedeutung besitzen:

| X | $R^1$ | $R^2$ | Y |
|---|---|---|---|
| CH | H | $-OCH_3$ | O |
| " | " | $-OC_2H_5$ | " |
| " | " | $-OSC_4H_9$ | " |
| " | " | $-O-CH_2C_6H_5$ | " |
| " | " | $-O-CH_2-4ClC_6H_4$ | " |
| " | 5-Cl | $-OCH_3$ | " |
| " | " | $-OC_2H_5$ | " |
| " | " | $-O-CH_2C_6H_5$ | " |
| " | $5-NO_2$ | $-OCH_3$ | " |
| " | " | $-O-iC_3H_7$ | " |
| " | $5-CF_3$ | $-O-CH_3$ | " |
| " | " | $-O-CH_2C_6H_5$ | " |
| " | 4-Cl | $-O-C_2H_5$ | " |
| " | $5-NH_2$ | $-O-CH_3$ | " |
| " | $5\text{-NH-}\underset{\underset{O}{\|}}{C}\text{-CH}_3$ | $-O-CH_3$ | " |
| " | H | $-NH-4ClC_6H_4$ | " |
| " | " | $-NH-3CF_3C_6H_4$ | " |
| " | $5-NO_2$ | $-NH-3ClC_6H_4$ | " |
| " | " | $-NH-C_6H_5$ | " |
| " | $5-CF_3$ | $-NH-4CH_3C_6H_4$ | " |
| N | H | $-OCH_3$ | " |
| " | " | $-OC_2H_5$ | " |

| X | $R^1$ | $R^2$ | Y |
|---|---|---|---|
| N | " | $-O-nC_4H_9$ | O |
| " | " | $-O-CH_2C_6H_5$ | " |
| " | " | $-NH-4ClC_6H_4$ | " |
| " | " | $-NH-3,4Cl_2C_6H_3$ | " |
| " | 5-Cl | $-OCH_3$ | " |
| CH | H | $-NH-COOCH_3$ | " |
| " | -5-Cl | $-NH-COOC_2H_5$ | " |
| " | $-5,6-Cl_2$ | $-NH-COOCH_3$ | " |
| " | $-5-COOCH_3$ | $-NH-COOC_2H_5$ | " |
| " | $-5-SO_2NH_2$ | $-NH-COOC_2H_5$ | " |
| " | -H | $-NH-COOiC_3H_7$ | " |
| " | -H | $-NH-COOsC_4H_9$ | " |
| " | -5-Cl | $-NH-COOCH_2C_6H_5$ | " |
| " | $-5-NO_2$ | $-NH-COOCH_2C_6H_5$ | " |
| " | $-5-CF_3$ | $-NH-COOnC_3H_7$ | " |
| " | -H | $-OCH_3$ | NH |
| " | -5-Cl | $-OCH_3$ | " |
| " | $-5-NO_2$ | $-OC_2H_5$ | " |
| " | -H | $-OiC_3H_7$ | " |
| " | -H | $-OnC_4H_9$ | " |
| " | $-5,6(CH_3)_2$ | $-OsC_4H_9$ | " |
| " | -H | $-OCH_2C_6H_5$ | " |

| X | $R^1$ | $R^2$ | Y |
|---|---|---|---|
| " | -H | 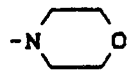 | " |
| " | -H | $-N(C_2H_5)_2$ | " |
| " | -5-Cl | 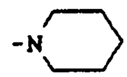 | " |
| " | $-5-CH_3$ | 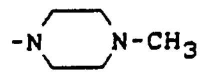 | " |
| " | -H | $-NH-C_6H_5$ | " |
| " | -5-Cl | $-NH-3,4\ Cl_2C_6H_32$ | " |
| " | $-5-NO_2$ | $-NH-4\ ClC_6H_4$ | " |
| " | -H | 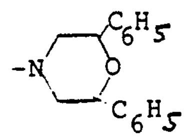 | NH |
| " | 5-Cl | 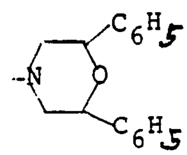 | NH |
| " | -H | 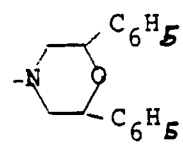 | O |
| " | -H | 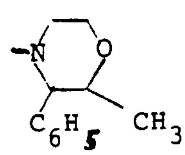 | NH |
| " | -H | 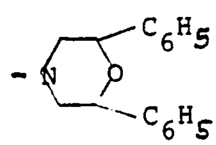 | O |
| N | -H | 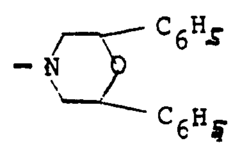 | NH |
| N | -H | 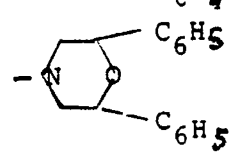 | O |

Setzt man bei Verfahren 3 a) als Verbindung der Formel (II) S-(2-Carbomethoxyphenyl)-thioglycolsäurecyclohexylester ein, so läßt sich das Verfahren durch folgendes Formelschema darstellen:

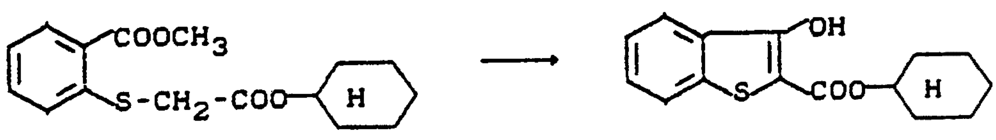

Die Verbindungen der Formel II sind teilweise bekannt. Sie können nach an sich bekannten Verfahren hergestellt werden (Katz et. al., J. Org. Chem. 18 (1953), S 1380; DE-OS 1 937 514; A.D. Dunn et. al., J. Heterocycl. Chem. 24 (1987), S 85).

Bevorzugt werden Verbindungen der Formel II eingesetzt, in denen $R^1$, $R^2$, X die bei den Verbindungen der Formel (I) angegebenen bevorzugten Bedeutungen besitzt und $R^6$ für Methyl oder Ethyl steht.

Im einzelnen seien folgende Verbindungen der Formel II genannt:
S-(2-Carbomethoxyphenyl)-thioglykolsäuremethylester, S-(2-Carbomethoxyphenyl)-thioglykolsäure-s-butylester, S-(2-Carbomethoxyphenylthioglykolsäureanilid, S-(2-Carboethoxyphenyl)-thioglykolsäure-(4-chloranilid), S-(2-Carbomethoxy-5-chlor)-thioglykolsäureethylester, S-(2-Carbomethoxyl-5-nitro)-thioglykolsäuremethylester, 5-(2-Carboethoxy-5-nitrophenyl)-thioglykolsäure-p-toluidid, S-(2-Carboethoxy-5-trifluormethylphenyl)-thioglykolsäure-methylester, 5-(2-Carbomethoxy-5,6-dichlorphenyl)-thioglykolsäurebenzylester, 2-Carbomethoxymethylthiopyridin-3-carbonsäuremethylester, 2-Carbobenzyloxymethylthio-pyridin-3-carbonsäuremethylester, 2-(N-Phenylcarbamoyl)-methylthiopyridin-3-carbonsäuremethylester.

Die Umsetzung erfolgt bei Temperaturen von 20 - 200 °C, bevorzugt bei 50 - 150 °C, besonders bevorzugt bei Siedetemperatur des Verdünnungsmittels.

Als Verdünnungsmittel kommen alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Alkohole wie Methanol, Ethanol, Isopropanol, Butanol, ferner Ether wie Diethyl- und Dibutylether, Glykoldimethylether und Diglykoldimethylether, Tetrahydrofuran und Dioxan, weiterhin Ketone, wie Aceton, Methylethyl-, Methylisopropyl- und Methylisobutylketon, außerdem Ester, wie Essigsäure-methylester und -ethylester, ferner Nitrile, wie z.B. Acetonitril und Propionitril, Benzonitril, Glutarsäuredinitril, darüber hinaus Amide, wie z.B. Dimethylformamid, Dimethylacetamid und N-Methylpyrrolidon, sowie Dimethylsulfoxid, Tetramethylensulfon und Hexamethylphosphorsäuretriamid.

Als Basen kommen anorganische und organische Basen in Frage. Als Basen seien genannt Alkali- und Erdalkalihydroxide, -carbonate, -hydrogencarbonate, -alkoholate, ferner Amine wie insbesondere tertiäre Amine z.B. Trimethylamin, Triethylamin, N-Methylmorpholin, Pyridin, Picoline, N-Ethylpyrrolidin, Diazabicyclo(4,3,0)-undecen(DBU), 1,4-Diazabicyclo(2,2,2)octan (DABCO), Diazabicyclo(3,2,0)nonen (DBN), Ethyl-diisopropylamin.

Die Verbindungen der Formeln II und die Basen werden im Verhältnis 1:1 bis 1:1,5 zueinander eingesetzt. Bevorzugt ist ein etwa äquimolares Verhältnis.

Nach erfolgter Umsetzung wird das Verdünnungsmittel zum Teil (zu ca. 50 %) abdestilliert, der Rest mit wässriger Säure versetzt und die Verbindungen der Formel I in an sich bekannter Weise isoliert, indem sie mit einem geeigneten Lösungsmittel, z.B. Ether oder Methylenchlorid, extrahiert werden. Die Verbindungen der Formel I können anschließend in üblicher Weise, z.B. chromatographisch, gereinigt werden.

Setzt man bei Verfahren 3 b als Verbindung der Formel III 2-Chlor-6-trifluormethyl-nicotinsäureethylester und als Verbindung der Formel IV Mercaptoessigsäure-m-chloranilid ein, so läßt sich der Reaktionsverlauf durch folgendes Reaktionsschema wiedergeben:

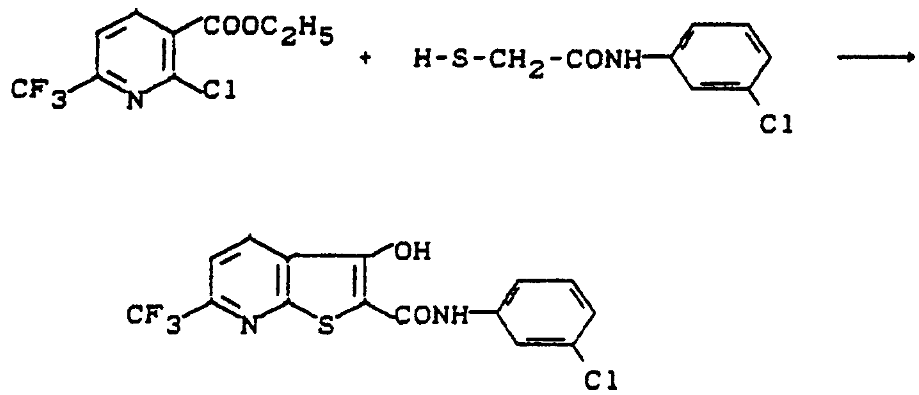

Bevorzugt werden Verbindungen der Formeln III und IV eingesetzt, in denen $R^1$, $R^2$, X und Y die bei den Verbindungen der Formel I angegebenen bevorzugten und besonders bevorzugten Bedeutungen haben und $R^6$ für Methyl oder Ethyl steht. Die Verbindungen der Formel III und IV sind bekannt oder lassen sich analog zu bekannten verfahren herstellen (DE-OS 1 937 514, Katz et. al., J. Org. Chem. 18 (1953), S. 1380, A. D. Dunn et. al., J. Heterocycl. Chem. 24 (1987) S. 85, J. Am. Chem. Soc. 69 (1947), S. 2914).

Im einzelnen seien folgende Verbindungen der Formel III genannt:
2-Chlor-5-nitro-benzoesäuremethylester, 2-Chlor-5-nitrobenzoesäureethylester, 2-Chlor-5-trifluormethyl-benzoesäuremethylester, 4-Chlorisophthalsäuredimethylester, 3-Carbomethoxy-4-chlordiphenylsulfon, 2-Chlornicotinsäuremethylester, 2,5-Dichlornicotinsäuremethylester, 2,6-Dichlornicotinsäureethylester.

Im einzelnen seien folgende Verbindungen der Formel IV genannt:
Thioglykolsäuremethyl-, -ethyl-, -iso-propyl-, -n-butyl-, -sek.butyl-, -tert.-butyl-, -benzyl- und -p-chlorbenzylester, Thioglykolsäureanilid, Thioglykolsäure-p-toluidid, Thioglykolsäure-p-chloranilid, Thioglykolsäure-3-trifluormethylanilid, Thioglykolsäure-3,4-dichloranilid, Thioglykolsäure-p-anisidid.

Verfahren 3b wird durchgeführt, indem man zunächst die Verbindungen der Formel IV in einem Lösungsmittel vorlegt, mit der in etwa äquimolaren Menge einer Base versetzt, und die Verbindung der Formel III in etwa äquimolarer Menge zufügt. Es ist auf dieser Stufe möglich, die offenkettige Verbindung der Formel II zu isolieren. Man kann jedoch auch ohne Isolierung der Verbindung der Formel II durch Zugabe weiterer in etwa äquimolarer Menge Base direkt die Verbindung der Formel I erhalten.

Als Basen und Lösungsmittel kommen die bei Verfahren 3 a genannten in Betracht. Neben den dort genannten Lösungsmitteln können auch aliphatische Alkohole verwendet werden.

Die Umsetzung erfolgt zwischen 0 und 200 °C, bevorzugt zwischen 10 und 100 °C, besonders bevorzugt bei Raumtemperatur oder der Siedetemperatur des eingesetzten Lösungsmittels. Zur Aufarbeitung wird das Reaktionsgemisch mit Wasser versetzt, angesäuert und abfiltriert oder extrahiert.

Setzt man bei Verfahren (5) als Verbindung der Formel (VI) S-(2-carbomethoxyphenyl)-thioglykolsäure-(N-Carboethoxy)-amid ein, so läßt sich das Verfahren durch folgendes Formelschema darstellen:

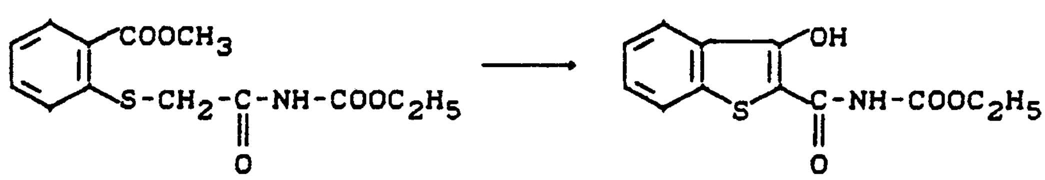

Bevorzugt werden Verbindungen der Formel (VI) eingesetzt, in denen $R^1$ und $R^5$ die bei den Verbindungen der Formel (I) angegebenen bevorzugten Bedeutungen besitzen und $R^6$ für Methyl oder Ethyl steht.

Im einzelnen seien folgende Verbindungen der Formel (VI) genannt:
S-(2-Carbomethoxyphenyl)-thioglykolsäure-(N-carboethoxy)-amid, S-(2-Carbomethoxy-5-chlorphenyl)-thioglykolsäure-(N-carbomethoxy)-amid, S-(2-Carbomethoxy-5-nitro-phenyl)-thioglykolsäure-(N-carbomethoxy)-amid, S-(2-Carboethoxy-5-trifluormethyl(phenyl)-thioglykolsäure-(N-carboethoxy)-amid, S-(2,5-Dicarbomethoxyphenyl)-thioglykolsäure-(N-carbomethoxy)-amid, S-(2-Carbomethoxyphenyl)-thioglykolsäure-(N-carbobenzyloxy)-amid, S-(2-Carbomethoxy-5-chlorphenyl)-thioglykolsäure-(N-carbo-sek.-butyloxy)-amid, S-(2-Carbomethoxy-5-methylphenyl)-thioglykolsäure-(N-carbomethoxy)-amid.

Die Umsetzung erfolgt bei Temperaturen von 20 - 200 °C, bevorzugt bei 50 - 150 °C, besonders

bevorzugt bei Siedetemperatur des Verdünnungsmittels.

Als Verdünnungsmittel kommen alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Alkohole wie Methanol, Ethanol, Isopropanol, Butanol, ferner Ether wie Diethyl- und Dibutylether, Glykoldimethylether und Diglykoldimethylether, Tetrahydrofuran und Dioxan, weiterhin Ketone, wie Aceton, Methylethyl-, Methylisopropyl- und Methylisobutylketon, außerdem Ester, wie Essigsäure-methylester und -ethylester, ferner Nitrile, wie z.B. Acetonitril und Propionitril, Benzonitril, Glutarsäuredinitril, darüber hinaus Amide, wie z.B. Dimethylformamid, Dimethylacetamid und N-Methylpyrrolidon, sowie Dimethylsulfoxid, Tetramethylensulfon und Hexamethylphosphorsäuretriamid.

Als Basen kommen anorganische und organische Basen in Frage. Als Basen seien genannt Alkali- und Erdalkalihydroxide, -carbonate, -hydrogencarbonate, -alkoholate, ferner Amine wie insbesondere tertiäre Amine z.B. Trimethylamin, Triethylamin, N-Methylmorpholin, Pyridin, Picoline, N-Ethylpyrrolidin, Diazabicyclo(4,3,0)-undecen(DBU), 1,4-Diazabicyclo(2,2,2)octan (DABCO), Diazabicyclo(3,2,0)nonen (DBN), Ethyl-diisopropylamin.

Die Verbindungen der Formeln II und die Basen werden im Verhältnis 1:1 bis 1:1,5 zueinander eingesetzt. Bevorzugt ist ein etwa äquimolares Verhältnis.

Nach erfolgter Umsetzung wird das Verdünnungsmittel zum Teil (zu ca. 50 %) abdestilliert, der Rest mit wässriger Säure versetzt und die Verbindungen der Formel I in an sich bekannter Weise isoliert, indem sie mit einem geeigneten Lösungsmittel, z.B. Ether oder Methylenchlorid, extrahiert werden. Die Verbindungen der Formel I können anschließend in üblicher Weise, z.B. chromatographisch, gereinigt werden.

Setzt man bei Verfahren (7) zur Herstellung der Verbindungen Formel (VI) als Verbindung der Formel (VII) S-(2-Ethoxycarbonyl-3-chlorphenyl )-thioglycolsäurechlorid und als Urethan der Formel (VIII) Cyclopropylurethan ein, läßt sich das Verfahren durch folgendes Formelschema wiedergeben:

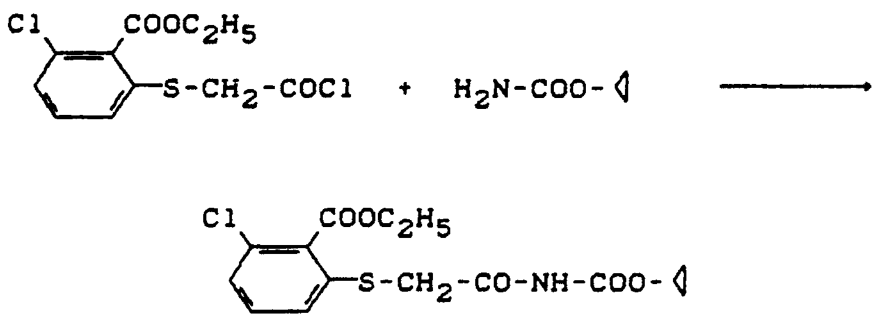

Die Verbindungen der Formel (VII) sind neu. Ihre Herstellung wird weiter unten beschrieben. Besonders genannt seien folgende Verbindungen der Formel (VII):

S-(2-Carbomethoxyphenyl)-thioglykolsäurechlorid, S-(4-Chlor-2-carbomethoxyphenyl)-thioglykolsäurechlorid, S-(4,5-Dichlor-2-carbomethoxyphenyl)-thioglykolsäurechlorid, S-(4-Nitro-2-carboethoxyphenyl)-thioglykolsäurechlorid, S-(4-Methyl-2-carbomethoxyphenyl)-thioglykolsäurechlorid, S-(4-Sulfamoyl-2-carboethoxyphenyl)-thioglykolsäurechlorid.

Urethane der Formel (VIII) sind bekannt. Insbesondere genannt seien:

Methylurethan, Ethylurethan, Propylurethan, i-Propylurethan, s-Butylurethan, Benzylurethan, 2-Chlorethylurethan, p-Chlorbenzylurethan, 3,4-Dichlorbenzylurethan.

Die Umsetzung erfolgt bevorzugt, indem äquimolare Mengen der Verbindungen (VII) und (VIII) zusammengegeben und erhitzt werden.

Die Reaktionstemperatur liegt zwischen 20-200 °C, bevorzugt zwischen 60-120 °C.

Es wird bei Normaldruck oder zwischen 1,5 und 10 bar gearbeitet.

Es kann auch in Gegenwart von Verdünnungsmitteln gearbeitet werden.

Setzt man bei Verfahren (8) zur Herstellung der Verbindungen der Formel (VII) S-(2-Methoxy-carbonyl-4-chlorphenyl)-thioglycolsäure und Thionylchlorid ein, läßt sich der Reaktionsverlauf wie folgt darstellen:

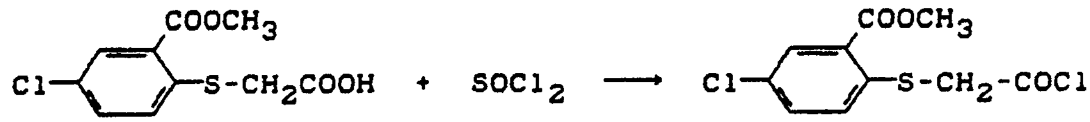

Verbindungen der Formel (IX) sind bekannt oder lassen sich nach bekannten Verfahren herstellen (vergl. Friedländer Liebigs Anm. Chem. 351 (1907), S. 390 - 420). Im einzelnen seien genannt: S-(2-carbomethoxyphenyl)-thioglykolsäure, S-(4-Chlor-2-carbomethoxyphenyl)-thioglykolsäure, S-(4,5-Dichlor-2-carboethoxyphenyl)-thioglykolsäure, S-(4-Nitro-2-carbomethoxyphenyl)-thioglykolsäure, S-(4,5-Dimethyl-2-carbomethoxyphenyl)-thioglykolsäure.

Die Umsetzung erfolgt, indem äquimolare Mengen Verbindung der Formel (IX) mit Thionylchlorid bei Temperaturen von 20° C bei 100° C und Drucken von Normaldruck bis 3 bar zur Reaktion gebracht werden. Gegebenenfalls kann auch in Gegenwart eines Verdünnungsmittels gearbeitet werden.

Die Wirkstoffe eignen sich bei günstiger Warmblütertoxizität zur Bekämpfung von pathogenen Endoparasiten die bei Menschen und in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien der Schädlinge sowie gegen resistente und normal sensible Arten wirksam. Durch die Bekämpfung der pathogenen Endoparasiten sollen Krankheit, Todesfälle und Leistungsminderungen (z.B. bei der Produktion von Fleisch, Milch, Wolle, Häuten, Eiern, Honig usw.) vermindert werden, so daß durch den Einsatz der Wirkstoffe eine wirtschaftlichere und einfachere Tierhaltung möglich ist. Zu den pathogenen Endoparasiten zählen Cestoden, Trematoden, Nematoden, Acantocephalen insbesondere:

Aus der Ordnung der Pseudophyllidea z.B.: Diphyllobothrium spp., Spirometra spp., Schistocephalus spp., Ligula spp., Bothridium spp., Diphlogonoporus spp..

Aus der Ordnung der Cyclophyllidea z.B.: Mesocestoides spp., Anoplocephala spp., Paranoplocephala spp., Moniezia spp., Thysanosomsa spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp..

Aus der Unterklasse der Monogenea z.B.: Gyrodactylus spp., Dactylogyrus spp., Polystoma spp..

Aus der Unterklasse der Digenea z.B.: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhlocoelum spp., Paramphistomum spp., Calicophoron spp-, Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp. Metorchis spp., Heterophyes spp., Metagonimus spp..

Aus der Ordnung der Enoplida z.B.: Trichuris spp., Capillaria spp., Trichomosoides spp., Trichinella spp..

Aus der Ordnung der Rhabditia z.B.: Micronema spp., Strongyloides spp..

Aus der Ordnung der Strongylida z.B.: Stronylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Bunostomum spp., Globocephalus spp., Syngamus spp., Cyathostoma spp., Metastrongylus spp., Dictyocaulus spp., Muellerius spp., protostrongylus spp., Neostrongylus spp., Cystocaulus spp., Pneumostrongylus spp., Spicocaulus spp., Elaphostrongylus spp. Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Marshallagia spp., Cooperia spp., Nematodirus spp., Hyostrongylus spp., Obeliscoides spp., Amidostomum spp., Ollulanus spp..

Aus der Ordnung der Oxyurida z.B.: Oxyuris spp., Enterobius spp., Passalurus spp., Syphacia spp., Aspiculuris spp., Heterakis spp..

Aus der Ordnung der Ascaridia z.B.: Ascaris spp., Toxascaris spp., Toxocara spp., Parascaris spp., Anisakis spp., Ascaridia spp..

Aus der Ordnung der Spirurida z.B.: Gnathostoma spp., Physaloptera spp., Thelazia spp., Gongylonema spp., Habronema spp., Parabronema spp., Draschia spp., Dracunculus spp..

Aus der Ordnung der Filariida z.B.: Stephanofilaria spp., Parafilaria spp., Setaria spp., Loa spp.,

Dirofilaria spp., Litomosoides spp., Brugia spp., Wuchereria spp., Onchocerca spp..

Aus der Ordnung dar Gigantorhynchida z.B.: Filicollis spp., Moniliformis spp., Macracanthorhynchus spp., Prosthenorchis spp..

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Süß- und Salzwasserfische wie z.B. Forellen, Karpfen, Aale, Reptilien, Insekten wie z.B. Honigbiene und Seidenraupe.

Zu Labor- und Versuchstieren gehören Mäuse, Ratten, Meerschweinchen, Goldhamster, Hunde und Katzen.

Zu den Hobbytieren gehören Hunde und Katzen.

Die Anwendung kann sowohl prophylaktisch als auch therapeutisch erfolgen.

Die Anwendung der Wirkstoffe erfolgt direkt oder in Form von geeigneten Zubereitungen enteral, parenteral, dermal, nasal, durch Behandlung der Umgebung oder mit Hilfe wirkstoffhaltiger Formkörper wie z.B. Streifen, Platten, Bänder, Halsbänder, Ohrmarken, Gliedmaßenbänder, Markierungsvorrichtungen.

Die enterale Anwendung der Wirkstoffe geschieht z.B. oral in Form von Pulver, Tabletten, Kapseln, Pasten, Tränken, Granulaten, oral applizierbaren Lösungen, Suspensionen und Emulsionen, Boli, medikiertem Futter oder Trinkwasser. Die dermale Anwendung geschieht z.B. in Form des Tauchens (Dippen), Sprühens (Sprayen) oder Aufgießens (pour-on and spot-on). Die parenterale Anwendung geschieht z.B. in Form der Injektion (intramusculär, subcutan, intravenös, intraperitoneal) oder durch Implantate.

Geeignete Zubereitungen sind:

Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;

Emulsionen und Suspension zur oralen oder dermalen Anwendung sowie zur Injektion; Halbfeste Zübereitungen;

Formulierungen bei denen der Wirkstoff in einer Salbengrundlage oder in einer Öl in Wasser oder Wasser in Öl Emulsionsgrundlage verarbeitet ist;

Feste Zubereitungen wie Pulver, Premixe oder Konzentrate, Granulate, Pellets, Tabletten, Boli, Kapseln; Aerosole und Inhalate, wirkstoffhaltige Formkörper.

Injektionslösungen werden intravenös, intramuskulär und subcutan verabreicht.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: Physiologisch verträgliche Lösungsmittel wie Wasser, Alkohole wie Ethanol, Butanol, Benzylakohol, Glycerin, Propylenglykol, Polyethylenglykole, N-Methyl-pyrrolidon, sowie Gemische derselben.

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzentrate werden wie oben bei den Injektionslösungen beschrieben hergestellt, wobei auf steriles Arbeiten verzichtet werden kann.

Lösungen zum Gebrauch auf der Haut werden aufgeträufelt, aufgestrichen, eingerieben, aufgespritzt oder aufgesprüht. Diese Lösungen werden wie oben bei den Injektionslösungen beschrieben hergestellt.

Es kann vorteilhaft sein, bei der Herstellung Verdikkungsmittel zuzufügen. Verdickungsmittel sind: Anorganische Verdickungsmittel wie Bentonite, kolloidale Kieselsäure, Aluminiummonostearat, organische Verdikkungsmittel wie Cellulosederivate, Polyvinylalkohole und deren Copolymere, Acrylate und Metacrylate.

Gele werden auf die Haut aufgetragen oder aufgestrichen oder in Körperhöhlen eingebracht. Gele werden hergestellt indem Lösungen, die wie bei den Injektionslösungen beschrieben hergestellt worden sind, mit soviel Verdickungsmittel versetzt werden, daß eine klare Masse mit salbenartiger Konsistenz entsteht. Als Verdickungsmittel werden die weiter oben angegebenen Verdickungsmittel eingesetzt.

Aufgieß-Formulierungen werden auf begrenzte Bereiche der Haut aufgegossen oder aufgespritzt, wobei der Wirkstoff die Haut durchdringt und systemisch wirkt.

Aufgieß.Formulierungen werden hergestellt, indem der Wirkstoff in geeigneten hautverträglichen Lösungsmitteln oder Lösungsmittelgemischen gelöst, suspendiert oder emulgiert wird. Gegebenenfalls werden

weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole, Glycole, Polyethylenglycole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmono-butylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2,2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfördernde Stoffe sind z.B.DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Novantisolsäure.

Haftmittel sind z.B. Cellulosederivate, Stärkederivate, Polyacrylate, natürliche Polymere wie Alginate, Gelatine.

Emulsionen können oral, dermal oder als Injektionen gewendet werden.

Emulsionen sind entweder vom Typ Wasser in Öl oder vom Typ Öl in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl/Caprinsäure-bigylcerid, Triglyceridgemisch mit Pflanzenfettsäuren der Kettenlänge $C_{8-12}$ oder anderen speziell ausgewählten natürlichen Fettsäuren, Partialglyceridgemische gesättigter oder ungesättigter eventuell auch hydroxylgruppenhaltiger Fettsäuren, Mono- und Diglyceride der $C_8/C_{10}$-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge $C_{16}$-$C_{18}$, Isopropylmyristat, Isopropylpalmitat, Capryl/Caprin säureester von gesättigten Fettalkoholen der Kettenlänge $C_{12}$-$C_{18}$, Isopropylstearat, Ölsäureoleylester, Ölsäuredecylester, Ethyloleat, Milchsäureethylester, wachsartige Fettsäureester wie künstliches Entenbürzeldrüsenfett, Dibutylphthalat, Adipinsäurediisopropylester, letzterem verwandte Estergemische u.a.

Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt:

Wasser, Alkohole wie z.B. Propylenglycol, Glycerin, Sorbitol und ihre Gemische.

Als Emulgatoren seien genannt: nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;

ampholytische Tenside wie Di-Na-N-lauryl-ß-iminodipropionat oder Lecithin;

anionaktive Tenside, wie Na-Laurylsulfat, Fettalkoholethersulfate, Mono/Dialkylpolyglykoletherorthophosphorsäureester-monoethanolaminsalz;

kationaktive Tenside wie Cetyltrimethylammoniumchlorid.

Als weitere Hilfsstoffe seien genannt: Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebene Tenside genannt.

Als weitere Hilfsstoffe seien die weiter oben angegebenen genannt.

Halbfeste Zubereitungen können oral oder dermal verabreicht werden. Sie unterscheiden sich von den

oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Als solche dienen anorganische und organische Stoffe Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungsund Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in den Zubereitungen auch in Mischung mit Synergisten oder mit anderen Wirkstoffen, die gegen pathogene Endoparasiten wirken, vorliegen. Solche Wirkstoffe sind z.B. L-2,3,5,6-Tetrahydro-6-phenylimidazothiazol, Benzimidazolcarbamate, Praziquantel, Pyrantel, Febantel.

Anwendungsfertige Zubereitungen enthalten den Wirkstoff in Konzentrationen von 10 ppm - 20 Gewichtsprozent, bevorzugt von 0,1 - 10 Gewichtsprozent.

Zubereitungen die vor Anwendung verdünnt werden, enthalten den Wirkstoff in Konzentrationen von 0,5 - 90 Gewichtsprozent, bevorzugt von 5 bis 50 Gewichtsprozent.

Im allgemeinen hat es sich als vorteilhaft erwiesen, Mengen von etwa 1 bis etwa 100 mg Wirkstoff je kg Körpergewicht pro Tag zur Erzielung wirksamer Ergebnisse zu verabreichen.

Beispiel A

In vivo Nematodentest

Trichostrongylus colubriformis / Schaf

Experimentell mit Trichostrongylus colubriformis infizierte Schafe wurden nach Ablauf der Präpatenzzeit des Parasiten behandelt. Die Wirkstoffe wurden als reiner Wirkstoff in Gelatinekapseln oral appliziert.

Der Wirkungsgrad wird dadurch bestimmt, daß man die mit dem Kot ausgeschiedenen Wurmeier vor und nach der Behandlung quantitativ auszählt.

Ein völliges Sistieren der Eiausscheidung nach der Behandlung bedeutet, daß die Würmer abgetrieben wurden oder so geschädigt sind, daß sie keine Eier mehr produzieren (Dosis effectiva).

Geprüfte Wirkstoffe und wirksame Dosierungen (Dosis effectiva) sind aus der nachfolgenden Tabelle ersichtlich.

| Wirkstoff Beispiel Nr. | Dosis effectiva in mg/kg |
|---|---|
| 3 | 10 |
| 10 | 10 |
| 65 | 10 |
| 20 | 10 |
| 54 | 5 |
| 55 | 5 |
| 63 a | 25 |
| 69 | 10 |
| 72 | 10 |
| 79 | 10 |
| 80 | 10 |
| 84 | 10 |
| 87 | 10 |
| 95 | 10 |

Beispiel B

In vivo Nematodentest

Haemonchus contortus / Schaf

Experimentell mit Haemonchus contortus infizierte Schafe wurden nach Ablauf der Präpatenzzeit des Parasiten behandelt. Die Wirkstoffe wurden als reiner Wirkstoff in Gelatinekapseln oral appliziert.

Der Wirkungsgrad wird dadurch bestimmt, daß man die mit dem Kot ausgeschiedenen Würmer vor und nach der Behandlung quantitativ auszählt.

Ein völliges Sistieren der Eiausscheidung nach der Behandlung bedeutet, daß die Würmer abgetrieben wurden oder so geschädigt sind, daß sie keine Eier mehr produzeiren (Dosis effectiva).

Geprüfte Wirkstoffe und wirksame Dosierungen (Dosis effectiva) sind aus der nachfolgenden Tabelle ersichtlich:

| Wirkstoff Beispiel Nr. | Dosis effectiva in mg/kg |
|---|---|
| 7 | 5 |
| 9 | 5 |
| 11 | 25 |
| 39 | 10 |
| 64 | 5 |
| 56 | 2,5 |
| 63 a | 5 |
| 71 | 5 |
| 79 | 10 |
| 81 | 5 |
| 95 | 5 |

Herstellungsbeispiele

Beispiel für Verfahren 3 a und 3 b

Herstellung von 3-Hydroxy-2-(3-chlorphenylcarbamoyl)thieno-[2,3b]-pyridin

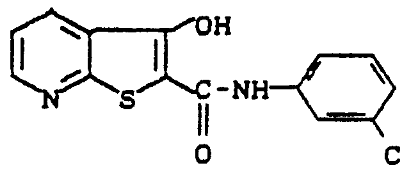

10,1 g (0,05 Mol) Mercaptoessigsäure-m-chloranilid (hergestellt nach J. Am. Chem. Soc. 69 (1947), S. 2314) werden bei Raumtemperatur in 100 ml Methanol vorgelegt und mit 2,7 g (0,05 Mol) Natriummethylat versetzt. Man rührt 10 Minuten bei Raumtemperatur nach und gibt dann 8,6 g (0,05 Mol) 2-Chlornicotinsäuremethylester zu. Nach einstündigem Nachrühren bei Raumtemperatur gibt man weitere 4,1 g (0,075 Mol) Natriummethylat zu und rührt über Nacht bei Raumtemperatur nach. Man gießt auf 500 ml Wasser, stellt mit Essigsäure sauer und saugt den Niederschlag ab, der durch Digerieren mit heißem Toluol gereinigt wird. Man erhält 4,7 g (31 % der Theorie) des Produktes vom Schmelzpunkt 247° C.

Herstellung von 3-Hydroxybenzothiophen-2-carbonsäureanilid

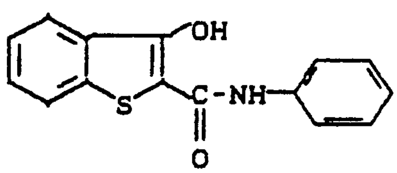

5,6 g (20,8 mmol) S-(2-Carbomethoxiphenyl)-thioglykolsäureanilid werden in 100 ml trockenem Toluol gelöst und 3,2 g (21 mmol) DBU zugefügt. Es wird zum Rückfluß erhitzt und dabei langsam Toluol abdestilliert. Nach vollständiger Umsetzung wird zwischen 100 ml verdünnter HCl-Lösung und Dichlormethan verteilt. Die dabei entstehende feste Zwischenphase wird abgesaugt und getrocknet.
Ausbeute: 3,23 g
Schmelzpunkt: 229° C (Zersetzung)

Herstellung von 3-Hydroxybenzothiophen-2-carbonsäurebenzylester

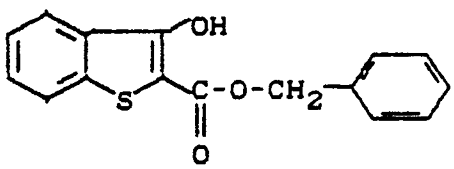

5 g (15,8 mmol) S-(2-Carbomethoxiphenyl)-thioglykolsäurebenzylester und 3 g (19,7 mmol) Diazabicycloundecen werden in 100 ml Toluol unter Rückfluß erhitzt. Dabei werden im Verlauf von einer Stunde etwa 50 ml Toluol abdestilliert. Nach Abkühlung wird auf 2N-HCl gegossen, mit $CH_2Cl_2$ extrahiert. Der Rückstand wird durch Filtration über Kieselgel mit Toluol als Laufmittel gereinigt.
Ausbeute: 2,6 g (58 % der Theorie)
Schmelzpunkt: 81° C

Herstellung von

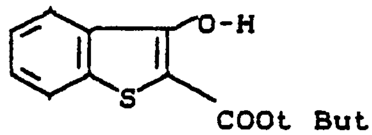

6,72 g (0,04 Mol) 2-Mercaptobenzoesäuremethylester und 6,02 g (0,04 Mol) Chloressigsäure-tert.-butylester werden in 30 ml trockenem Methanol vorgelegt. Zu diesen Mischungen wird bei Raumtemperatur eine Lösung von 4,32 g (0,08 Mol) Natriummethylat in 30 ml trockenem Methanol langsam zugetropft und bis zur vollständigen Umsetzung gerührt. Danach wird im Vakuum eingedampft. Der Rückstand wird zwischen Wasser und Dichlormethan verteilt, die organische Phase abgetrennt, mit $Na_2SO_4$ getrocknet und im Vakuum eingedampft.
Ausbeute: 9,6 g; Schmelzpunkt: 163° C.

Analog werden folgende Verbindungen hergestellt:

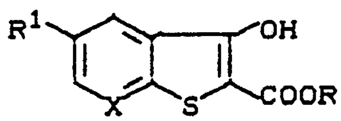

| Bsp. Nr. | X | $R^1$ | R | Schmp. [°C] |
|---|---|---|---|---|
| 1 | N | H | $CH_3$ | 144 |
| 2 | N | H | $C_2H_5$ | 62 |
| 3 | CH | $NO_2$ | $CH_3$ | 215 |
| 4 | CH | $NO_2$ | $-CH_2-C_6H_5$ | 158 |
| 5 | CH | $NO_2$ | $-C_2H_5$ | 176 |
| 6 | CH | $CF_3$ | $-CH_3$ | 121 |

| Bsp. Nr. | X | $R^1$ | R | Schmp. [°C] |
|---|---|---|---|---|
| 7 | CH | H | $-CH_3$ | 104 |
| 8 | CH | H | $-i-C_3H_7$ | $^1$HNMR($CDCl_3$) δ[ppm]:7,92(d,1H), 7,72 (d,1H), 7,5 (t,1H), 7,4, (t,1H), 5,3, (m,1H), 1,4, (d,6H) |
| 9 | CH | H | $-s-C_4H_9$ | |
| 10 | CH | H | $-CH_2-C_6H_6$ | 81 |
| 11 | CH | H | $-CH_2-CH_2-C_6H_5$ | 106 |
| 12 | CH | H | $-(CH_2)_3-C_6H_5$ | $^1$HNMR($CDCl_3$) δ[ppm]:7,95(d,1H), 7,75 (d,1H), 7,5 (t,1H), 7,4, (t,1H), 7,1-7,35 (m,5H), 4,38 (t,2H), 2,29 (t,2H), 2,0-2,2 (m,2H) |
| 13 | CH | H | 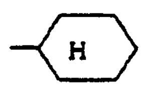 | 69 |
| 14 | CH | H | 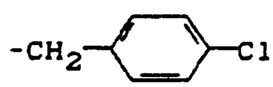 | 124 |
| 15 | CH | H | 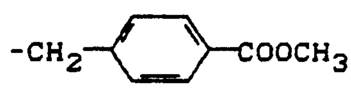 | 104 |
| 16 | CH | H | 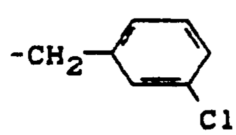 | 134 |
| 17 | CH | H | $n-C_4H_9$ | 77 |
| 18 | CH | H | $i-C_4H_9$ | 81 |
| 19 | CH | H | 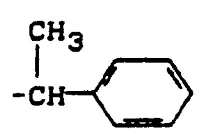 | 88 |

Fortsetzung

| Bsp. Nr. | X | $R^1$ | $R^3$ | Schmp. [°C] |
|---|---|---|---|---|
| 20 | CH | -Br | $-CH_3$ | 174 |
| 21 | CH | -Br | $-C_2H_5$ | 99 |
| 22 | CH | $-SO_2NH_2$ | $-CH_3$ | >260 |
| 23 | CH | $-CF_3$ | $-C_2H_5$ | 100 |
| 24 | CH | $-NO_2$ | $s-C_4H_9$ | 119 |
| 25 | CH | $-NO_2$ | $i-C_3H_7$ | 209 |
| 26 | CH | $-COOCH_3$ | $-CH_3CH_3$ | 171 |
| 27 | CH | $-NO_2$ | $-n-C_4H_9$ | 117 |
| 28 | CH | $-COOC_2H_5$ | $-C_2H_5$ | 82 |
| 28a | CH | 5-Cl | $-CH_3$ | 141 |
| 28b | CH | 5-Cl | $-C_2H_5$ | 103 |
| 28c | CH | 6-Cl | $-CH_2$-Phenyl | 94 |
| 28d | CH | $5-CH_3$ | $-CH_3$ | 74 |
| 28e | CH | $5-CH_3$ | $-C_2H_5$ | 64 |
| 28f | CH | $5-CH_3$ | $-CH_2$-Phenyl | 79 |
| 28g | CH | -H | 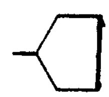 | 207 |
| 28h | CH | 5-Cl | 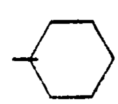 | > 250 °C |

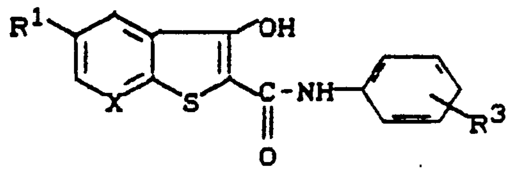

| Bsp. Nr. | X | $R^1$ | $R^3$ | Schmp. [°C] |
|---|---|---|---|---|
| 29 | N | -H | -H | 241 |
| 30 | N | -H | -3-Cl | 247 |
| 31 | CH | $-NO_2$ | -H | 239 |
| 32 | CH | $-NO_2$ | -3-Cl | 230 |
| 33 | CH | $-NO_2$ | -4-Cl | 257 |
| 34 | CH | $-NO_2$ | -4-F | 248-254 |
| 35 | CH | $-NO_2$ | $-4-OCH_3$ | 228 |
| 36 | CH | $-NO_2$ | $-3-CH_3$ | 189 |
| 37 | CH | $-NO_2$ | $-4-CH_3$ | 223 |
| 38 | CH | $-CF_3$ | -H | 197 |
| 39 | CH | $-NO_2$ | $-3-CF_3$ | 202 |
| 40 | N | -H | $-4-CH_3$ | 259 |
| 41 | N | -H | -4-Cl | 270 |
| 42 | CH | -H | -3-Cl | 221 |
| 43 | CH | -H | -H | 229 |
| 44 | CH | -H | $-4-SCF_3$ | 213 |
| 45 | CH | -H | $-4-CF_3$ | 200 |
| 46 | CH | -H | -4-Cl | 230 |
| 47 | CH | -Br | $-3-CF_3$ | 178 |
| 48 | CH | -H | $-3-CH_3$ | 193 |

| Bsp. Nr. | X | $R^1$ | $R^3$ | Schmp. [°C] |
|---|---|---|---|---|
| 49 | CH | 5-$COOCH_3$ | 4-$CH_3$ | 208 |
| 50 | CH | 5-$COOCH_3$ | 4-$OCH_3$ | 194 |
| 51 | CH | 5-$COOCH_3$ | -H | 233 |
| 52 | CH | 5-$COOCH_3$ | -3-$CH_3$ | 179 |
| 53 | CH | 5-$COOCH_3$ | -4-Cl | 135 |
| 54 | CH | 5-$COOCH_3$ | -3-$CF_3$ | 196 |
| 55 | CH | 5-$COOCH_3$ | -3-Cl | 223-227 |
| 55a | CH | H | 4-O-$CH_3$ | 187 |

Herstellung von 3-Hydroxy-5-nitro-benzothiophen-2-carbonsäure-N-(carboethoxy)-amid

(Beispiel 56)

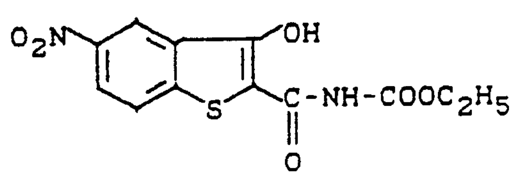

6,8 g (0,02 Mol) S-(2-Carbomethoxy-5-nitrophenyl)-thioglykolsäure-N-(carboethoxy)-amid werden zu einer Lösung von 0,98 g (0,025 Mol) Natrium in 100 ml Ethanol gegeben und die erhaltene Mischung 12 Stunden bei Raumtemperatur gerührt. Dann wird in 500 ml Wasser unter Rühren gegos sen, mit 10 %iger Salzsäure auf pH 4 gestellt und der ausgefallene Feststoff abgesaugt, auf der Nutsche mit Wasser gewaschen und getrocknet. Das Produkt ist praktisch analysenrein.
Ausbeute 4,5 g (73 % d. Th.); Schmp. 259° C.

Das als Ausgangsmaterial benötigte S-(2-Carbomethoxy-5-nitrophenyl)-thioglykolsäure-N-(carboethoxy)-amid wird wie folgt erhalten:

7,3 g (0,025 Mol) S-(2-Carbomethoxy-5-nitrophenyl)-thioglykolsäurechlorid und 2,5 g (0,0275 Mol) Ethylurethan werden 6 Stunden auf 80 - 90° C erhitzt. Nach dem Abkühlen wird die erstarrte Masse im Mörser zerkleinert, mit 500 ml Wasser verrührt, die Suspension abgesaugt, mit Wasser gewaschen, in 300 ml Ethanol aufgekocht, abgekühlt und erneut abgesaugt. Das Produkt wird auf der Nutsche gewaschen und getrocknet. Die Ausbeute beträgt 4 g (47 % d. Th.) bei einer Reinheit von 97,5 % (HFC).

Nach dem Verfahren 5 werden die folgenden Beispiele hergestellt:

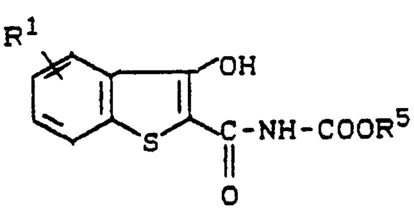

| Bsp.-Nr. | $R^1$ | $R^5$ | Fp °C |
|---|---|---|---|
| 57 | -H | $-CH_3$ | > 270 |
| 58 | -H | $-n-C_4H_8$ | 113° C (Zers.) |
| 59 | -H | $-CH_2-C_6H_5$ | 154° C (Zers.) |
| 60 | $-5-COOCH_3$ | $-CH_2-C_6H_5$ | 158° C |
| 61 | $-5-COOCH_3$ | $-n-C_4H_9$ | 144° C (Zers.) |
| 62 | $-5-COOCH_3$ | $-C_2H_5$ | 173° C (Zers.) |
| 63 | $-5-COOCH_3$ | $-CH_3$ | 251° C |

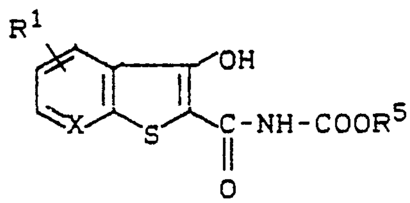

| Bsp. Nr. | X | $R^1$ | $R^5$ | Schmp. [°C] |
|---|---|---|---|---|
| 63a | CH | $5-NO_2$ | $-C_2H_5$ | 259 |
| 63b | CH | H | $-C_2H_5$ | 260 |
| 63c | CH | 5-Cl | $-CH_3$ | 250 (Zers.) |
| 63d | CH | $5-CH_3$ | $-CH_3$ | >265 |
| 63e | CH | $5-CH_3$ | $C_2H_5$ | >265 |
| 63f | CH | $5-CH_3$ | $n-C_4H_9$ | 92 |
| 63g | CH | $5-CH_3$ | $-CH_2$-Phenyl | 133 |
| 63h | N | H | $C_2H_5$ | >250 |

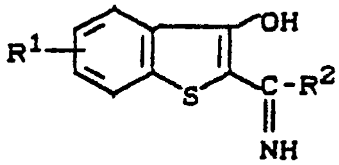

| Bsp. Nr. | $R^1$ | $R^2$ | Schmp. |
|---|---|---|---|
| 64 | H | $-OCH_3$ | 195° C |
| 65 | " | $-O-sC_4H_9$ | 196° C |
| 66 | " | $-OC_2H_5$ | 186° C |
| 67 | " | $-O-iC_3H_7$ | 234° C |
| 68 | " | $-O-nC_4H_9$ | 181° C |
| 69 | " | $-NH-C_6H_5$ | 224° C |
| 70 | " | $-NH-4-ClC_6H_4$ | 265-270° C |
| 71 | " | $-N(C_2H_5)_2$ | 142° C |
| 72 | " | $-NH-3CF_3C_6H_4$ | 227° C |
| 73 | " | $-NH-3ClC_6H_4$ | 209° C |
| 74 | " | $-NH-4COOCH_3C_6H_4$ | >270° C |
| 75 | " | $-NH-CH_2-C_6H_5$ | 224° C |
| 76 | " | $-NH-CH_2-3CF_3C_6H_4$ | 237° C |
| 77 | " | 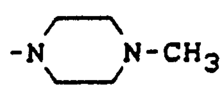 | 140° C |
| 78 | " | 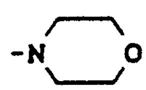 | 147° C |

| Bsp. Nr. | $R^1$ | $R^2$ | Schmp. |
|---|---|---|---|
| 79 | H | 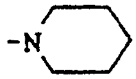 | 161° C |
| 80 | " | 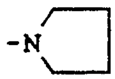 | 248° C |
| 81 | " | 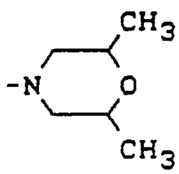 | 172° C |
| 82 | " | $-NH-CH_2-4-ClC_6H_4$ | 221° C |

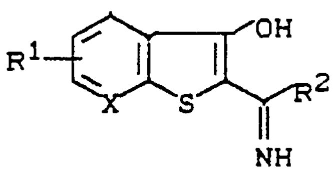

| Bsp. Nr. | X | $R^1$ | $R^2$ | Schmp. [°C] |
|---|---|---|---|---|
| 83 | CH | 6-Cl | $O-CH_3$ | 213 |
| 84 | CH | 6-Cl | $O-(CH_2)_2-CH_3$ | 238 |
| 85 | CH | 6-Cl | $-O-i-C_3H_7$ | 220 |
| 86 | CH | 6-Cl | $-O-S-C_4H_9$ | 218 |
| 87 | CH | $5-CH_3$ | $-O-CH_3$ | 243 |
| 88 | CH | $5-CH_3$ | $-O-(CH_2)_2-CH_3$ | 224 |
| 89 | CH | $5-CH_3$ | $-O-n-C_4H_9$ | 196 |

| Bsp. Nr. | X | $R^1$ | $R^2$ | Schmp. [°C] |
|---|---|---|---|---|
| 90 | CH | $5-CH_3$ | $-O-S-C_4H_9$ | 171 |
| 91 | CH | 5-Cl | $-O-CH_3$ | 248 |
| 92 | CH | 5-Cl | $-O-C_2H_5$ | 232 |
| 93 | CH | 5-Cl | $-O-n-C_3H_7$ | 224 |
| 94 | CH | 5-Cl | $-O-i-C_3H_7$ | 207 |
| 95 | CH | 5-Cl | $-O-s-C_4H_9$ | 129 |
| 96 | CH | 6-Cl | $-O-C_2H_5$ | 224 |
| 97 | CH | $5-CH_3$ | $-O-C_2H_5$ | 234 |
| 98 | CH | 5-Cl | $-O-CH_2-C_6H_4$ | 94 |
| 99 | CH | H | $-O-CH_2-C_5H_9$ | 207 |
| 100 | CH | 5-Cl | $-O-CH_3-C_5H_9$ | 187 |
| 101 | CH | $5-CH_3$ | $-\underset{H}{N}-2-Cl-C_6H_4$ | 184 |
| 102 | CH | $5-CH_3$ | $-\underset{H}{N}-3-Cl-C_6H_4$ | 207 |
| 103 | CH | $5-CH_3$ | $-\underset{H}{N}-4-Cl-C_6H_4$ | 261 |
| 104 | CH | 5-Cl | $-\underset{H}{N}-2-Cl-C_6H_4$ | 258 |
| 105 | CH | 5-Cl | $-\underset{H}{N}-2-Cl-C_6H_4$ | 208 |
| 106 | CH | 5-Cl | $-\underset{H}{N}-4-Cl-C_6H_4$ | >260 |
| 107 | CH | 6-Cl | $-\underset{H}{N}-2-Cl-C_6H_4$ | 214 |
| 108 | CH | 6-Cl | $-\underset{H}{N}-3-Cl-C_6H_4$ | 203 |

| Bsp. Nr. | X | $R^1$ | $R^2$ | Schmp. [°C] |
|---|---|---|---|---|
| 109 | CH | 6-Cl | $-\underset{H}{N}-4-Cl-C_6H_4$ | 241 |
| 110 | CH | 6-Cl | $-\underset{H}{N}-3-CH_3-C_6H_4$ | 218 |
| 111 | CH | 6-Cl | $-\underset{H}{N}-4-CH_3-C_6H_4$ | 253 |
| 112 | CH | 6-Cl | $-\underset{H}{N}-3-CH_3-C_6H_4$ | 197 |
| 113 | CH | H | $-\underset{H}{N}-CH(CH_3)-C_6H_4$ | 161 |
| 114 | CH | $5-CH_3$ | $-NH-3-CH_3-C_6H_4$ | 177 |
| 115 | CH | $5-CH_3$ | $-NH-2-CH_3-C_6H_4$ | 194 |
| 116 | CH | 5-Cl | $-NH-2-CH_3-C_6H_4$ | 253 |
| 117 | CH | 5-Cl | $-NH-3-CH_3-C_6H_4$ | 217 |
| 118 | CH | 5-Cl | $-NH-4-CH_3-C_6H_4$ | 282 |
| 119 | CH | 5-Cl | $-NH-3-CF_3-C_6H_4$ | 216 |
| 120 | CH | 5-Cl | $-NH-4-OCH_3-C_6H_4$ | 274 |
| 121 | CH | H | $-NH-4-F-C_6H_4$ | 284 |
| 122 | CH | 5-Cl | 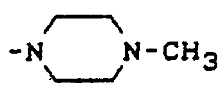 | 151 |
| 123 | CH | 5-Cl | $NH-CH(CH_3)-C_6H_4$ | 193 |
| 124 | CH | $5-CH_3$ | $-NH-3-CF_3-C_6H_4$ | 189 |
| 125 | CH | $5-CH_3$ | $-NH-4-OCF_3-C_6H_4$ | 222 |
| 126 | CH | $5-CH_3$ | $-NH-4-F-C_6H_4$ | 265 |

| Bsp. Nr. | X | $R^1$ | $R^2$ | Schmp. [°C] |
|---|---|---|---|---|
| 127 | CH | 5-$CH_3$ | -NH-CH($CH_3$)-$C_6H_5$ | 87 |
| 128 | CH | 5-$CH_3$ | 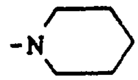 | 188 |
| 129 | CH | 5-Cl | 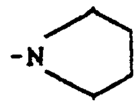 | >265 |
| 130 | CH | 5-$CH_3$ | 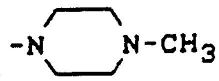 | 177 |
| 131 | CH | 5-$CH_3$ | 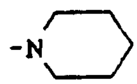 | 188 |
| 132 | CH | 5-$CH_3$ | 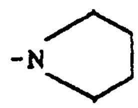 | 269 |
| 133 | CH | 5-$CH_3$ | 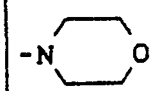 | 203 |
| 134 | CH | 5-$CH_3$ | 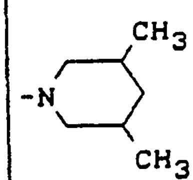 | 168 |
| 135 | N | H | -$OCH_3$ | >250 |
| 136 | N | H | -$OC_2H_5$ | 124 |
| 137 | N | H | -O-n-$C_3H_7$ | 184 |
| 138 | N | H | -O-i-$C_3H_7$ | 169 |

| Bsp. Nr. | X | $R^1$ | $R^2$ | Schmp. [°C] |
|---|---|---|---|---|
| 139 | N | H | $-O-n-C_4H_7$ | 188 |
| 140 | N | H | $-O-S-C_4H_9$ | 173 |
| 141 | N | H | $-NH-3-Cl-C_6H_4$ | >250 |
| 142 | N | H | $-NH-4-Cl-C_6H_4$ | >250 |
| 143 | N | H | $-NH-4-CH_3-C_6H_4$ | >260 |
| 144 | N | H | $-NH-3-CH_3C_6H_4$ | 251 |
| 145 | N | H | $-NH-3-CH_3-C_6H_4$ | 257 |
| 146 | N | H | $-NH-4-F-C_6H_4$ | 269 |
| 147 | CH | $5-CH_3$ | $-NH-4-CH_3C_6H_4$ | 227 |

Beispiele zur Herstellung der Ausgangsprodukte der Formel (II):

Herstellung von 5-(2-Carbomethoxyphenyl)-thioglykolsäurebenzylester

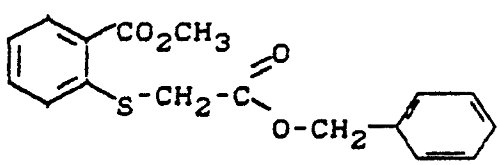

5 g (0,02 mol) S-(2-Carbomethoxiphenyl)-thioglykolsäurechlorid, gelöst in 50 ml abs. $CHCl_3$ werden unter Kühlung zu einer Mischung aus 2,2 g (0.02 mol) Benzylalkohol, 2,8 ml (0,02 mol) $Et_3N$ und 50 ml abs. $CHCl_3$ zu getropft. Es wird noch eine Stunde nachgerührt, dann die ganze Mischung auf eine 5-prozentige wässrige $NaH_2PO_4$-Lösung gegossen. Die organische Phase wird abgetrennt, die wässrige Phase nochmals mit $CHCl_3$ extrahiert, mit $Na_2SO_4$ getrocknet und eingedampft. Zur Reinigung wird über Kieselgel mit Dichlormethan filtriert.
Ausbeute: 3,5g Öl (54% der Theorie)

Das Produkt kann auch roh in die nächste Stufe eingesetzt werden.

$^1H$-NMR($CDCl_3$):
7,95 ppm (dd, 1H, $H_{arom}$),
7,1 - 7,4 ppm (m, 8H, $H_{arom}$),
5.15 ppm (s, 2H, $-CH_2-Ar$),
3,9 ppm (s, 3H, $O-CH_3$),
3,77 ppm (s, 2H, $-SCH_2-COO-$)

Herstellung von S-(2-Carbomethoxyphenyl)-thioglykolsäureanilid

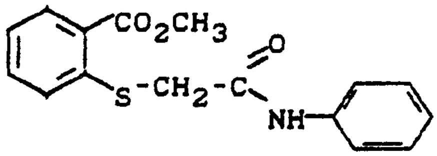

1,85 ml (20 mmol) Anilin werden in 20 ml abs. Chloroform vorgelegt und unter Eiskühlung 2,44 g (10 mmol) S-(2-Carbomethoxyphenyl)-thioglykolsäurechlorid, gelöst in 10 ml abs. Chloroform zugetropft. Es wird eine Stunde nachgerührt, dann mit 100 ml Wasser versetzt. Die organische Phase wird abgetrennt und nacheinander mit 5-prozentiger $H_2SO_4$, Wasser und $NaHCO_3$-Lösung gewaschen. Es wird mit $Na_2SO_4$ getrocknet und eingedampft.
Ausbeute: 3,57 g
Gehalt: 90,8 % (GC/MS)
$^1$H-NMR($CDCl_3$):
8,7 ppm (5, 1H, NH),
8,0 ppm (dd, 1H, $H_{arom}$),
7,0 - 7,5 ppm (m, 8H, $H_{arom}$),
3,95 ppm (s, 3H, $-OCH_3$),
3,87 ppm (s, 2H, $-SCH_2$-CONH)

Analog werden hergestellt:

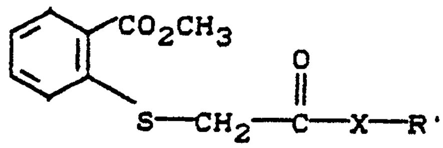

| | X | $R^1$ | physikalische Daten $^1H$-NMR ($CDCl_3$); δ[ppm] |
|---|---|---|---|
| d | O | 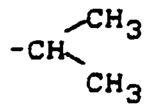 | 7,99 (d,1H), 7,35-7,5 (m,2H), 7,2 (t,1H), 4,95-5,1 (m,1H), 3,92 (s,3H), 3,68 (s,2H), 1,22 (d,6H) |
| e | O | 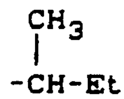 | 7,98 (d,1H), 7,35-7,5 (m, 2H), 7,19 (t,1H), 4,8-4,95 (m,1H), 3,92 (s,3H), 3,71 (s,2H), 1,42-1,68 (m,2H), 1,2 (d,3H), 0,85 (t,3H) |
| f | O | 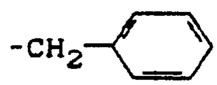 | 7,98 (d, 1H), 7,22-742 (m, 7H), 7,2 (t, 1H), 5,16 (s,2H), 3,9 (s,3H), 3,75 (s,2H) |
| g | O | 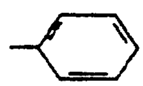 | Schmelzpunkt: 75° C |
| h | O | 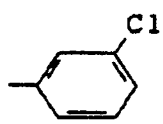 | Schmelzpunkt: 45° C |
| i | O | 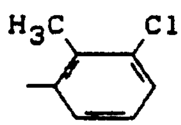 | Schmelzpunkt: 86° C |
| k | O | 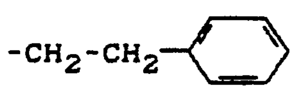 | 7,98 (d,1H), 7,1-7,45 (m,8H), 4,35 (t,2H), 3,92 (s,3H), 3,69 (s,2H), 2,92 (t,2H) |

| | X | $R^1$ | physikalische Daten $^1H$-NMR ($CDCl_3$); δ[ppm] |
|---|---|---|---|
| l | O | 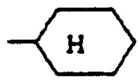 | 7,98 (d,1H), 7,4-7,5 (m,2H), 7,19 (t,1H), 4,75-4,85 (m,1H), 3,92 (s,3H), 3,7 (s,2H), 1,15-1,87 (m,10H) |
| m | O | 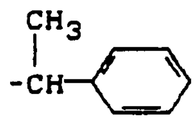 | 7,98 (d,1H), 7,21-7,39 (m,7H), 7,19 (t,1H), 5,91 (q,1H), 3,92 (s,3H), 3,73 (s,2H), 1,52 (d,3H) |
| n | O | 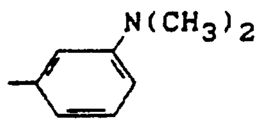 | 8,0 (d,1H), 7,45-7,08 (m,2H), 7,12-7,28 (m,2H), 6,55 (dd,1H), 6,38 (dd,1H), 6,3 (t,1H), 3,93 (s,5H), 2,90 (s,6H) |
| o | O | Et | 7,98 (d,1H), 7,38-7,51 (m,2H), 7,22 (t,1H), 4,20 (q,2H), 3,92 (s,3H), 3,71 (s,2H), 1,25 (t,3H) |
| p | NH | 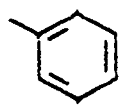 | Schmelzpunkt: 133° C |
| q | NH | 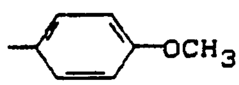 | Schmelzpunkt: 94° C |
| r | NH | 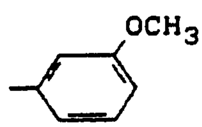 | Schmelzpunkt: 110° C |
| s | NH | 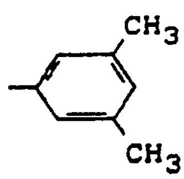 | Schmelzpunkt: 122° C |

| | X | $R^1$ | physikalische Daten $^1H$-NMR ($CDCl_3$); δ[ppm] |
|---|---|---|---|
| t | NH | 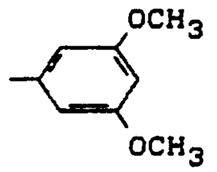 | 8,65 (s,1H), 8,02 (d,1H), 7,5 (t,1H), 7,32 (d,1H), 7,25 (t,1H), 6,74 (d,2H), 6,22 (t,1H), 3,96 (s,3H), 3,8 (s,2H), 3,75 (s,6H) |
| u | NH | 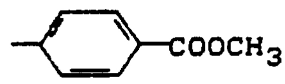 | (gemessen in $DMSO^6$-D): 7,92 (t,3H), 7,74 (d,2H), 7,18-7,32 (m,1H), 7,52-7,6 (m,2H), 3,96 (s,2H), 3,85 (s,3H), 3,82 (s,3H) |

Herstellung von S-(2-Carbomethoxyphenyl)-thioglykolsäurechlorid

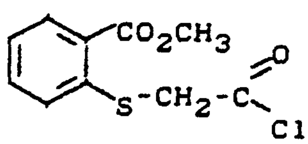

2,26 ml (10 mmol) S-(2-Carbomethoxyphenyl)-thioglykolsäure[1)] werden in 20 ml Thionylchlorid eingetragen und drei Stunden unter Rückfluß erhitzt. Anschließend wird im Vakuum eingedampft und zweimal mit trockenem Toluol nachdestilliert. Zuletzt wird an der Ölpumpe getrocknet. Der Rückstand wird ohne weitere Reinigung weiter verwendet. Rotes Öl, das langsam zu einer wachsartigen Masse erstarrt.

2,26 ml (10 mmol) S-(2-Carbomethoxyphenyl)-thioglykolsäure 1) werden in 20 ml Thionylchlorid eingetragen und drei Stunden unter Rückfluß erhitzt. Anschließend wird im Vakuum eingedampft und zweimal mit trockenem Toluol nachdestilliert. Zuletzt wird an der Ölpumpe getrocknet. Der Rückstand wird ohne weitere Reinigung weiter verwendet. Rotes Öl, das langsam zu einer wachsartigen Masse erstarrt.

## Ansprüche

1. Verwendung von 3-Hydroxybenzothiophenen der Formel (I)

1) erhalten gemäß Friedländer, Liebigs Ann. Chem. 351, (1907) 390 - 420

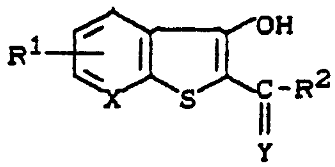

(I)

in welcher
X für =CH- oder =N- steht,
Y für O oder =NH steht,
$R^1$ für einen oder mehrere, gleiche oder verschiedene der Reste Wasserstoff, Alkyl, Alkoxy, Alkylthio, Halogenalkyl, Halogenalkoxy, Halogenalkylthio, Alkylendioxy, Halogenalkylendioxy, Halogen, CN, $NO_2$, $NH_2$, Alkylamino, Dialkylamino, Alkylcarbonyl, Carbalkoxy, Alkylsulfonyl, Arylsulfonyl, Sulfamoyl, Alkylsulfamoyl, Dialkylsulfamoyl, Aryl, Aryloxy, Arylthio, die ihrerseits wieder substituiert sein können, steht,
$R^2$ für gegebenenfalls substituiertes Alkoxy, Cycloalkoxy oder den Rest-$NR^3R^4$ steht,
$R^3$ für Wasserstoff oder Alkyl steht,
$R^4$ für Alkyl, Aralkyl oder carbocyclische, heterocyclische aromatische Reste oder den Rest -$COOR^5$ steht,
$R^3$ und $R^4$ gemeinsam mit dem angrenzenden Stickstoffatom für einen 5- oder 6-gliedrigen Heterocyclus stehen, der als weitere Heteroatome O oder N enthalten kann und gegebenenfalls durch $C_1$-$C_4$-Alkyl, $C_1$-$C_4$-Hydroxyalkyl, $C_1$-$C_4$-Halogenalkyl, $C_1$-$C_4$-Alkoxyalkyl gegebenenfalls substituiertes Aryl substituiert ist.
$R^5$ für Alkyl, Cycloalkyl, Aralkyl oder Aryl, die ihrerseits wieder substituiert sein können, steht,
zur Bekämpfung von Endoparasiten in der Medizin und Tiermedizin.

2. 3-Hydroxybenzothiophene der Formel (I)

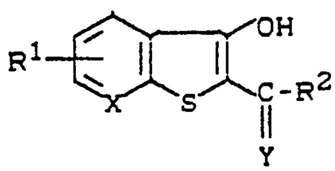

(I)

in welcher
X für =N- steht,
Y für =O oder =NH steht,
$R^1$ für einen oder mehrere, gleiche oder verschiedene der Reste Wasserstoff, Alkyl, Alkoxy, Alkylthio, Halogenalkyl, Halogenalkoxy, Halogenalkylthio, Alkylendioxy, Halogenalkylendioxy, Halogen, CN, $NO_2$, $NH_2$, Alkylamino, Dialkylamino, Alkylcarbonyl, Carbalkoxy, Alkylsulfonyl, Arylsulfonyl, Sulfamoyl, Alkylsulfamoyl, Dialkylsulfamoyl, Aryl, Aryloxy, Arylthio, die ihrerseits wieder substituiert sein können, steht,
$R^2$ für gegebenenfalls substituiertes $C_{2-6}$-Alkoxy, Cycloalkoxy oder den Rest -$NR^3R^4$ steht,
$R^3$ für Wasserstoff oder Alkyl steht,
$R^4$ für Alkyl, Aralkyl, carbocyclische oder heterocyclische aromatische Reste steht,
$R^3$ und $R^4$ gemeinsam mit dem angrenzenden Stickstoffatom für einen 5- oder 6-gliedrigen Heterocyclus stehen, der als weitere Heteroatome O oder N enthalten kann und gegebenenfalls durch $C_1$-$C_4$-Alkyl, $C_1$-$C_4$-Hydroxyalkyl, $C_1$-$C_4$-Halogenalkyl, $C_1$-$C_4$-Alkoxyalkyl gegebenenfalls substituiertes Aryl substituiert ist.

3. Verfahren zur Herstellung der neuen 3-Hydroxybenzothiophene der Formel I

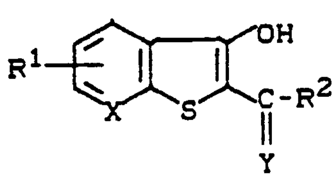

(I)

in welcher
X für =N- steht,
Y für =O oder =NH steht,
$R^1$ für einen oder mehrere, gleiche oder verschiedene der Reste Wasserstoff, Alkyl, Alkoxy, Alkylthio, Halogenalkyl, Halogenalkoxy, Halogenalkylthio, Alkylendioxy, Halogenalkylendioxy, Halogen, CN, $NO_2$, $NH_2$,

Alkylamino, Dialkylamino, Alkylcarbonyl, Carbalkoxy, Alkylsulfonyl, Arylsulfonyl, Sulfamoyl, Alkylsulfamoyl, Dialkylsulfamoyl, Aryl, Aryloxy, Arylthio, die ihrerseits wieder substituiert sein können, steht,
$R^2$ für gegebenenfalls substituiertes $C_{2-6}$-Alkoxy, Cycloalkoxy oder den Rest $-NR^3R^4$ steht,
$R^3$ für Wasserstoff oder Alkyl steht,
$R^4$ für Alkyl, Aralkyl, carbocyclische oder heterocyclische aromatische Reste steht,
$R^3$ und $R^4$ gemeinsam mit dem angrenzenden Stickstoffatom für einen 5- oder 6-gliedrigen Heterocyclus stehen, der als weitere Heteroatome O oder N enthalten kann und gegebenenfalls durch $C_1$-$C_4$-Alkyl, $C_1$-$C_4$-Hydroxyalkyl, $C_1$-$C_4$-Halogenalkyl, $C_1$-$C_4$-Alkoxyalkyl gegebenenfalls substituiertes Aryl substituiert ist, dadurch gekennzeichnet, daß man

a) Verbindungen der Formel (II)

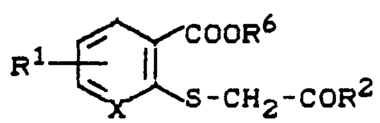

(II)

in welcher
X, $R^1$, $R^2$ die oben angegebene Bedeutung haben,
$R^6$ für $C_{1-4}$-Alkyl steht,
in Gegenwart einer Base erhitzt, oder indem man

b) Verbindungen der Formel (III)

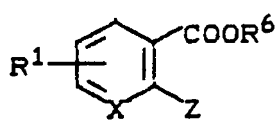

(III)

in welcher
X, $R^1$, $R^6$ die oben angegebene Bedeutung besitzen,
Z für Halogen steht,
mit Verbindungen der Formel (IV)

$H\text{-}S\text{-}CH_2\text{-}C(=O)\text{-}R^2$ (IV)

in welcher
$R^2$ die oben angegebene Bedeutung besitzt, in Gegenwart einer Base umsetzt.

4. 3-Hydroxybenzothiophene der Formel (V)

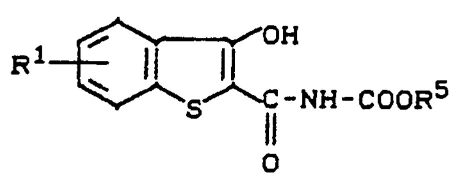

(V)

in welcher
$R^1$ für einen oder mehrere, gleiche oder verschiedene der Reste Wasserstoff, Alkyl, Alkoxy, Alkylthio, Halogenalkyl, Halogenalkoxy, Halogenalkylthio, Alkylendioxy, Halogenalkylendioxy, Halogen, CN, $NO_2$, $NH_2$, Alkylamino, Dialkylamino, Alkylcarbonyl, Carbalkoxy, Alkylsulfonyl, Arylsulfonyl, Sulfamoyl, Alkylsulfamoyl, Dialkylsulfamoyl, Aryl, Aryloxy, Arylthio, die ihrerseits wieder substituiert sein können, steht,
$R^5$ für Alkyl, Cycloalkyl, Aralkyl oder Aryl, die ihrerseits wieder substituiert sein können, steht.

5. Verfahren zur Herstellung der neuen 3-Hydroxybenzothiophene der Formel (V)

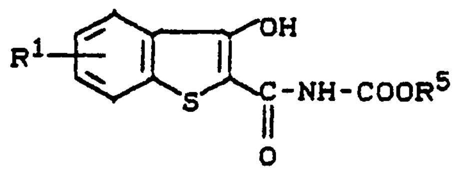

(V)

in welcher
$R^1$ für einen oder mehrere, gleiche oder verschiedene der Reste Wasserstoff, Alkyl, Alkoxy, Alkylthio, Halogenalkyl, Halogenalkoxy, Halogenalkylthio, Alkylendioxy, Halogenalkylendioxy, Halogen, CN, $NO_2$, $NH_2$, Alkylamino, Dialkylamino, Alkylcarbonyl, Carbalkoxy, Alkylsulfonyl, Arylsulfonyl, Sulfamoyl, Alkylsulfamoyl, Dialkylsulfamoyl, Aryl, Aryloxy, Arylthio, die ihrerseits wieder substituiert sein können, steht,
$R^5$ für Alkyl, Cycloalkyl, Aralkyl oder Aryl, die ihrerseits wieder substituiert sein können, steht,
dadurch gekennzeichnet, daß man
Verbindungen der Formel (VI)

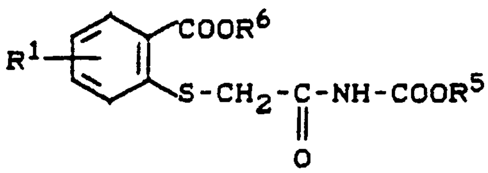

(VI)

in welcher
$R^1$ und $R^5$ die oben angegebene Bedeutung haben,
$R^6$ für $C_1$-$C_4$-Alkyl steht,
in Gegenwart einer Base erhitzt.

6. Verbindungen der Formel (VI)

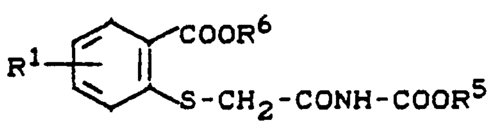

(VI)

in welcher
$R^1$, $R^5$, $R^6$ die in Anspruch 5 angegebene Bedeutung haben.

7. Verfahren zur Herstellung der Verbindungen der Formel (VI) gemäß Anspruch 6, dadurch gekennzeichnet, daß man Säurechloride der Formel (VII)

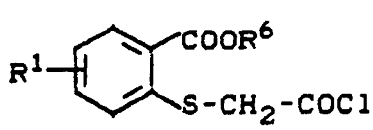

(VII)

in welcher
$R^1$, $R^6$ die in Anspruch 5 angegebene Bedeutung haben,
mit Urethanen der Formel (VIII)
$H_2N$-$COOR^5$ (VIII)
in welcher
$R^5$ die in Anspruch 5 angegebene Bedeutung hat,
umsetzt.

8. Verbindungen der Formel (VII)

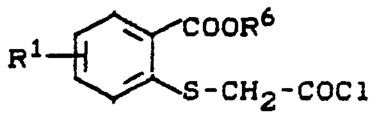

(VII)

in welcher
$R^1$ und $R^6$ die in Anspruch 6 angegebene Bedeutung haben.

9. Verfahren zur Herstellung der Verbindungen der Formel VII gemäß Anspruch 8, dadurch gekennzeichnet, daß man 3-(2-Carboalkoxyphenyl)-thioglycolsäure der Formel (IX)

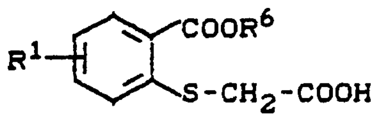

(IX)

in welcher
$R^1$ und $R^6$ die in Aspruch 6 angegebene Bedeutung haben,
mit Thionylchlorid, Phosphortrichlorid oder Phosgen umsetzt.

10. Endoparasitizide Mittel, gekennzeichnet durch einen Gehalt an mindestens einem 3-Hydroxybenzothiophen der Formel (I) gemäß Anspruch 1.

11. Verfahren zur Herstellung von endoparasitiziden Mitteln, dadurch gekennzeichnet, daß man 3-Hydroxybenzothiophene der Formel (I) gemäß Anspruch 1 mit Streckmitteln und/oder oberflächenaktiven Mitteln vermischt.

12. Verwendung von 3-Hydroxybenzothiophenen der Formel (I) gemäß Anspruch 1 zur Herstellung von endoparasitiziden Mitteln.

Europäisches Patentamt

# EUROPÄISCHER RECHERCHENBERICHT

Nummer der Anmeldung

EP 90110764.9

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.⁵) |
|---|---|---|---|
| D,A | EP - A1 - 0 160 408 (MERCK & CO) * Ansprüche * | 1,4,10 | C 07 D 333/70<br>C 07 D 495/04<br>A 61 K 31/38<br>A 61 K 31/44<br>C 07 C 323/62 |
| A | DE - C - 229 067 (R. LESSER) * Gesamt * | 6,8 | |
| A | DE - C - 825 268 (H. TIMMLER et al.) * Gesamt * | 6,8 | |
| A | CHEMICAL ABSTRACTS, Band 107, Nr. 21, 23. November 1987 Columbus, Ohio, USA A.D. DUNN et al. "Nucleophihc displacements in pyridine rings" Seite 756, Spalte 1, Zusammenfassung-Nr. 198 232w & J. Heterocylc. Chem. 1987, 24(1)-85.9. | 2,3 | |

RECHERCHIERTE SACHGEBIETE (Int. Cl.⁵)

C 07 D 333/00
C 07 D 495/00
A 61 K 31/00
C 07 C 323/00

Der vorliegende Recherchenbericht wurde für alle Patentansprüche erstellt.

| Recherchenort | Abschlußdatum der Recherche | Prüfer |
|---|---|---|
| WIEN | 13-09-1990 | HOFBAUER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument